# EUROPÄISCHE PATENTANMELDUNG

(11) **EP 3 348 326 A1**
(43) Veröffentlichungstag der Anmeldung: **18.07.2018**
(21) Anmeldenummer: 17209837.8
(22) Anmeldetag: 21.12.2017
(51) Int. Cl.: B01J 31/00, B01J 31/06, B01J 31/16, B01J 31/22, C12N 1/20

(54) **GANZZELL-BASIERTE BIOHYBRIDKATALYSATOR-SYSTEME**

(30) Priorität: 22.12.2016 DE 102016125516
(71) Anmelder: Rheinisch-Westfälische Technische Hochschule (RWTH) Aachen, 52062 Aachen (DE)
(72) Erfinder: Schwaneberg, Ulrich, 4728 KELMIS-HERGENRATH (BE); Okuda, Jun, 52074 Aachen (DE); Sauer, Daniel, 52064 Aachen (DE); Arlt, Marcus, 52064 Aachen (DE); Zhu, Leilei, 52074 Aachen (DE); Bocola, Marco, 52066 Aachen (DE); Grimm, Alexander, 52064 Aachen (DE); Mertens, Alan, 4700 EUPEN (BE)
(74) Vertreter: Eisenführ Speiser

(57) **Zusammenfassung**

Die vorliegende Erfindung betrifft ein Biohybridkatalysator-System, umfassend oder bestehend aus (a) einer intakten, nicht-menschlichen Zelle und (b) einem oder mehreren künstlichen Metalloenzym(en) bestehend aus (b1) einem Proteingerüst und (b2) einem an das Proteingerüst angebundenen, katalytisch aktiven Metallkomplex, wobei das/die Metalloenzym(e) in der Zellmembran verankert und/oder an der Zelloberfläche der Zelle angebunden ist/sind. Gemäß weiteren Aspekten betrifft die vorliegende Erfindung ebenfalls ein Verfahren zur Herstellung eines solchen Biohybridkatalysator-Systems und die Verwendung eines solchen Biohybridkatalysator-Systems als Katalysator zur Katalyse von chemischen Reaktionen.

SEQUENCE LISTING

<110>  Rheinisch-Westfälische Technische Hochschule Aachen (RWTH)

<120>  Ganzzell-basierte Biohybridkatalysator-Systeme

<130>  RM 5185-02EP

<150>  DE102016125516.5
<151>  2016-12-22

<160>  18

<170>  PatentIn version 3.5

<210>  1
<211>  1809
<212>  DNA
<213>  artificial

<220>
<223>  artificial

<400>  1
atggcgcgtt ccaaaactgc tcagccaaaa cactcactgc gtaaaatcgc agttgtagta      60

gccacagcgg ttagcggcat gtctgtttat gcacaggcac tgaaagaagt tcagtttaaa     120

gccggtactg acagcctgtt ccagactggt tttgacttta gcgattcgtt ggatgatgac     180

ggtgtttact cttatcgcct gaccggtctt gcgcgttctg ccaatgccca gcagaaaggg     240

tcagaagagc agcgttatgc tattgcaccg gcgttcacct ggcgtccgga tgataaaacc     300

aattttacct tcctttctta cttccagaac gagccggaaa ccggttatta cggctggttg     360

ccgaaagagg gaaccgttga gccgctgccg aacggtaagc gtctgccgac agactttaat     420

gaaggggcga agaacaacac ctattctcgt aatgagaaga tggtcggcta cagcttcgat     480

cacgaattta acgacacctt tactgtgcgt cagaacctgc gctttgctga aaacaaaacc     540

tcgcaaaaca gcgtttatgg ttacggcgtc tgctccgatc cggcgaatgc ttacagcaaa     600

cagtgtgcgg cattagcgcc agcggataaa ggccattatc tggcacgtaa atacgtcgtt     660

gatgatgaga agctgcaaaa cttctccgtt gatacccagt tgcagagcaa gtttgccact     720

ggcgatatcg accacaccct gctgaccggt gtcgacttta tgcgtatgcg taatgacatc     780

aacgcctggt ttggttacga cgactctgtg ccactgctca atctgtacaa tccggtgaat     840

accgatttcg acttcaatgc caaagatccg gcaaactccg gcccttaccg cattctgaat     900

aaacagaaac aaacgggcgt ttatgttcag gatcaggcgc agtgggataa agtgctggtc     960

accctaggcg gtcgttatga ctgggcagat caagaatctc ttaaccgcgt tgccgggacg    1020

accgataaac gtgatgacaa acagtttacc tggcgtggtg gtgttaacta cctgtttgat    1080

aatggtgtaa caccttactt cagctatagc gaatcgtttt tcccttcttc gcaagttggg    1140

aaggaaaacc tgtacttcca gggggatggt aatattttcg caccgtctaa aggtaagcag    1200

tatgaagtcg gcgtgaaata tgtaccggaa gatcgtccga ttgtagttac tggtgccgtg 1260

tataatctca cttgcaccaa cgttctgatg gcggaccctg agggtgagaa tctgtacttc 1320

caagggtcct tcttctcggt tgaaggtggc gagatccgcg cacgtggcgt agaaatcgaa 1380

gcgaaagcgg cgctgtcggc gagtgttaac gtagtcggtt cttatactta caccgatgcg 1440

gaatacacca ccgatactac ctataaaggc aatacgcctg cacaggtgcc aaaacacatg 1500

gcttcgttgt gggctgacta caccttcttt gacggtccgc tttcaggtct gacgctgggc 1560

accggtggtc gttatactgg ctccagttat ggtgatccgg ctaactcctt taaagtggga 1620

agttatacgg tcgtggatgc gttagtacgt tatgatctgg cgcgagtcgg catggctggc 1680

tccaacgtgg cgctgcatgt taacaacctg ttcgatcgtg aatacgtcgc cagctgcttt 1740

aacacttatg gctgcttctg gggcgcagaa cgtcaggtcg ttgcaaccgc aaccttccgt 1800

ttctaataa 1809

<210> 2
<211> 601
<212> PRT
<213> artificial

<220>
<223> artificial

<400> 2

Met Ala Arg Ser Lys Thr Ala Gln Pro Lys His Ser Leu Arg Lys Ile
1               5                   10                  15

Ala Val Val Val Ala Thr Ala Val Ser Gly Met Ser Val Tyr Ala Gln
            20                  25                  30

Ala Leu Lys Glu Val Gln Phe Lys Ala Gly Thr Asp Ser Leu Phe Gln
        35                  40                  45

Thr Gly Phe Asp Phe Ser Asp Ser Leu Asp Asp Asp Gly Val Tyr Ser
    50                  55                  60

Tyr Arg Leu Thr Gly Leu Ala Arg Ser Ala Asn Ala Gln Gln Lys Gly
65                  70                  75                  80

Ser Glu Glu Gln Arg Tyr Ala Ile Ala Pro Ala Phe Thr Trp Arg Pro
                85                  90                  95

Asp Asp Lys Thr Asn Phe Thr Phe Leu Ser Tyr Phe Gln Asn Glu Pro
            100                 105                 110

Glu Thr Gly Tyr Tyr Gly Trp Leu Pro Lys Glu Gly Thr Val Glu Pro

115                 120                 125

Leu Pro Asn Gly Lys Arg Leu Pro Thr Asp Phe Asn Glu Gly Ala Lys
    130                 135                 140

Asn Asn Thr Tyr Ser Arg Asn Glu Lys Met Val Gly Tyr Ser Phe Asp
145                 150                 155                 160

His Glu Phe Asn Asp Thr Phe Thr Val Arg Gln Asn Leu Arg Phe Ala
                165                 170                 175

Glu Asn Lys Thr Ser Gln Asn Ser Val Tyr Gly Tyr Gly Val Cys Ser
            180                 185                 190

Asp Pro Ala Asn Ala Tyr Ser Lys Gln Cys Ala Ala Leu Ala Pro Ala
        195                 200                 205

Asp Lys Gly His Tyr Leu Ala Arg Lys Tyr Val Val Asp Asp Glu Lys
    210                 215                 220

Leu Gln Asn Phe Ser Val Asp Thr Gln Leu Gln Ser Lys Phe Ala Thr
225                 230                 235                 240

Gly Asp Ile Asp His Thr Leu Leu Thr Gly Val Asp Phe Met Arg Met
                245                 250                 255

Arg Asn Asp Ile Asn Ala Trp Phe Gly Tyr Asp Asp Ser Val Pro Leu
            260                 265                 270

Leu Asn Leu Tyr Asn Pro Val Asn Thr Asp Phe Asp Phe Asn Ala Lys
        275                 280                 285

Asp Pro Ala Asn Ser Gly Pro Tyr Arg Ile Leu Asn Lys Gln Lys Gln
    290                 295                 300

Thr Gly Val Tyr Val Gln Asp Gln Ala Gln Trp Asp Lys Val Leu Val
305                 310                 315                 320

Thr Leu Gly Gly Arg Tyr Asp Trp Ala Asp Gln Glu Ser Leu Asn Arg
                325                 330                 335

Val Ala Gly Thr Thr Asp Lys Arg Asp Asp Lys Gln Phe Thr Trp Arg
            340                 345                 350

Gly Gly Val Asn Tyr Leu Phe Asp Asn Gly Val Thr Pro Tyr Phe Ser
        355                 360                 365

Tyr Ser Glu Ser Phe Phe Pro Ser Ser Gln Val Gly Lys Glu Asn Leu
    370                 375                 380

Tyr Phe Gln Gly Asp Gly Asn Ile Phe Ala Pro Ser Lys Gly Lys Gln
385                 390                 395                 400

Tyr Glu Val Gly Val Lys Tyr Val Pro Glu Asp Arg Pro Ile Val Val
                405                 410                 415

Thr Gly Ala Val Tyr Asn Leu Thr Cys Thr Asn Val Leu Met Ala Asp
            420                 425                 430

Pro Glu Gly Glu Asn Leu Tyr Phe Gln Gly Ser Phe Phe Ser Val Glu
        435                 440                 445

Gly Gly Glu Ile Arg Ala Arg Gly Val Glu Ile Glu Ala Lys Ala Ala
    450                 455                 460

Leu Ser Ala Ser Val Asn Val Val Gly Ser Tyr Thr Tyr Thr Asp Ala
465                 470                 475                 480

Glu Tyr Thr Thr Asp Thr Thr Tyr Lys Gly Asn Thr Pro Ala Gln Val
                485                 490                 495

Pro Lys His Met Ala Ser Leu Trp Ala Asp Tyr Thr Phe Phe Asp Gly
            500                 505                 510

Pro Leu Ser Gly Leu Thr Leu Gly Thr Gly Gly Arg Tyr Thr Gly Ser
        515                 520                 525

Ser Tyr Gly Asp Pro Ala Asn Ser Phe Lys Val Gly Ser Tyr Thr Val
    530                 535                 540

Val Asp Ala Leu Val Arg Tyr Asp Leu Ala Arg Val Gly Met Ala Gly
545                 550                 555                 560

Ser Asn Val Ala Leu His Val Asn Asn Leu Phe Asp Arg Glu Tyr Val
                565                 570                 575

Ala Ser Cys Phe Asn Thr Tyr Gly Cys Phe Trp Gly Ala Glu Arg Gln
            580                 585                 590

Val Val Ala Thr Ala Thr Phe Arg Phe
        595                 600

<210>  3
<211>  1800
<212>  DNA

<213>  artificial

<220>
<223>  artificial

<400>  3
atggcgcgtt ccaaaactgc tcagccaaaa cactcactgc gtaaaatcgc agttgtagta      60

gccacagcgg ttagcggcat gtctgtttat gcacaggcac tgaaagaagt tcagtttaaa     120

gccggtactg acagcctgtt ccagactggt tttgacttta gcgattcgtt ggatgatgac     180

ggtgtttact cttatcgcct gaccggtctt gcgcgttctg ccaatgccca gcagaaaggg     240

tcagaagagc agcgttatgc tattgcaccg gcgttcacct ggcgtccgga tgataaaacc     300

aattttacct tcctttctta cttccagaac gagccggaaa ccggttatta cggctggttg     360

ccgaaagagg gaaccgttga gccgctgccg aacggtaagc gtctgccgac agactttaat     420

gaaggggcga agaacaacac ctattctcgt aatgagaaga tggtcggcta cagcttcgat     480

cacgaattta acgacacctt tactgtgcgt cagaacctgc gctttgctga aaacaaaacc     540

tcgcaaaaca gcgtttatgg ttacggcgtc tgctccgatc cggcgaatgc ttacagcaaa     600

cagtgtgcgg cattagcgcc agcggataaa ggccattatc tggcacgtaa atacgtcgtt     660

gatgatgaga agctgcaaaa cttctccgtt gatacccagt tgcagagcaa gtttgccact     720

ggcgatatcg accacaccct gctgaccggt gtcgacttta tgcgtatgcg taatgacatc     780

aacgcctggt ttggttacga cgactctgtg ccactgctca atctgtacaa tccgtcttct     840

caccaccacc accaccacgg ttcttctgtg aataccgatt tcgacttcaa tgccaaagat     900

ccggcaaact ccggccctta ccgcattctg aataaacaga aacaaacggg cgtttatgtt     960

caggatcagg cgcagtggga taaagtgctg gtcaccctag gcggtcgtta tgactgggca    1020

gatcaagaat ctcttaaccg cgttgccggg acgaccgata aacgtgatga caaacagttt    1080

acctggcgtg gtggtgttaa ctacctgttt gataatggtg taacacctta cttcagctat    1140

agcgaatcgt ttgaaccttc ttcgcaagtt gggaaggatg gtaatatttt cgcaccgtct    1200

aaaggtaagc agtatgaagt cggcgtgaaa tatgtaccgg aagatcgtcc gattgtagtt    1260

actggtgccg tgtataatct cactaaaacc aacaacctga tggcggaccc tgagggttcc    1320

ttcttctcgg ttgaaggtgg cgagatccgc gcacgtggcg tagaaatcga agcgaaagcg    1380

gcgctgtcgg cgagtgttaa cgtagtcggt tcttatactt acaccgatgc ggaatacacc    1440

accgatacta cctataaagg caatacgcct gcacaggtgc caaaacacat ggcttcgttg    1500

tgggctgact acaccttctt tgacggtccg ctttcaggtc tgacgctggg caccggtggt    1560

cgttatactg gctccagtta tggtgatccg gctaactcct ttaaagtggg aagttatacg    1620

gtcgtggatg cgttagtacg ttatgatctg gcgcgagtcg gcatggctgg ctccaacgtg    1680

gcgctgcatg ttaacaacct gttcgatcgt gaatacgtcg ccagctgctt taacacttat    1740

ggctgcttct ggggcgcaga acgtcaggtc gttgcaaccg caaccttccg tttctaataa     1800

<210>  4
<211>  598
<212>  PRT
<213>  artificial

<220>
<223>  artificial

<400>  4

Met Ala Arg Ser Lys Thr Ala Gln Pro Lys His Ser Leu Arg Lys Ile
1               5                   10                  15

Ala Val Val Val Ala Thr Ala Val Ser Gly Met Ser Val Tyr Ala Gln
            20                  25                  30

Ala Leu Lys Glu Val Gln Phe Lys Ala Gly Thr Asp Ser Leu Phe Gln
        35                  40                  45

Thr Gly Phe Asp Phe Ser Asp Ser Leu Asp Asp Asp Gly Val Tyr Ser
    50                  55                  60

Tyr Arg Leu Thr Gly Leu Ala Arg Ser Ala Asn Ala Gln Gln Lys Gly
65                  70                  75                  80

Ser Glu Glu Gln Arg Tyr Ala Ile Ala Pro Ala Phe Thr Trp Arg Pro
                85                  90                  95

Asp Asp Lys Thr Asn Phe Thr Phe Leu Ser Tyr Phe Gln Asn Glu Pro
            100                 105                 110

Glu Thr Gly Tyr Tyr Gly Trp Leu Pro Lys Glu Gly Thr Val Glu Pro
        115                 120                 125

Leu Pro Asn Gly Lys Arg Leu Pro Thr Asp Phe Asn Glu Gly Ala Lys
    130                 135                 140

Asn Asn Thr Tyr Ser Arg Asn Glu Lys Met Val Gly Tyr Ser Phe Asp
145                 150                 155                 160

His Glu Phe Asn Asp Thr Phe Thr Val Arg Gln Asn Leu Arg Phe Ala
                165                 170                 175

Glu Asn Lys Thr Ser Gln Asn Ser Val Tyr Gly Tyr Gly Val Cys Ser
            180                 185                 190

Asp Pro Ala Asn Ala Tyr Ser Lys Gln Cys Ala Ala Leu Ala Pro Ala

195                 200                 205

Asp Lys Gly His Tyr Leu Ala Arg Lys Tyr Val Val Asp Asp Glu Lys
    210                 215                 220

Leu Gln Asn Phe Ser Val Asp Thr Gln Leu Gln Ser Lys Phe Ala Thr
225                 230                 235                 240

Gly Asp Ile Asp His Thr Leu Leu Thr Gly Val Asp Phe Met Arg Met
                245                 250                 255

Arg Asn Asp Ile Asn Ala Trp Phe Gly Tyr Asp Asp Ser Val Pro Leu
            260                 265                 270

Leu Asn Leu Tyr Asn Pro Ser Ser His His His His His His Gly Ser
        275                 280                 285

Ser Val Asn Thr Asp Phe Asp Phe Asn Ala Lys Asp Pro Ala Asn Ser
    290                 295                 300

Gly Pro Tyr Arg Ile Leu Asn Lys Gln Lys Gln Thr Gly Val Tyr Val
305                 310                 315                 320

Gln Asp Gln Ala Gln Trp Asp Lys Val Leu Val Thr Leu Gly Gly Arg
                325                 330                 335

Tyr Asp Trp Ala Asp Gln Glu Ser Leu Asn Arg Val Ala Gly Thr Thr
            340                 345                 350

Asp Lys Arg Asp Asp Lys Gln Phe Thr Trp Arg Gly Gly Val Asn Tyr
        355                 360                 365

Leu Phe Asp Asn Gly Val Thr Pro Tyr Phe Ser Tyr Ser Glu Ser Phe
    370                 375                 380

Glu Pro Ser Ser Gln Val Gly Lys Asp Gly Asn Ile Phe Ala Pro Ser
385                 390                 395                 400

Lys Gly Lys Gln Tyr Glu Val Gly Val Lys Tyr Val Pro Glu Asp Arg
                405                 410                 415

Pro Ile Val Val Thr Gly Ala Val Tyr Asn Leu Thr Lys Thr Asn Asn
            420                 425                 430

Leu Met Ala Asp Pro Glu Gly Ser Phe Phe Ser Val Glu Gly Gly Glu
        435                 440                 445

Ile Arg Ala Arg Gly Val Glu Ile Glu Ala Lys Ala Ala Leu Ser Ala
    450                 455                 460

Ser Val Asn Val Val Gly Ser Tyr Thr Tyr Thr Asp Ala Glu Tyr Thr
465                 470                 475                 480

Thr Asp Thr Thr Tyr Lys Gly Asn Thr Pro Ala Gln Val Pro Lys His
                485                 490                 495

Met Ala Ser Leu Trp Ala Asp Tyr Thr Phe Phe Asp Gly Pro Leu Ser
            500                 505                 510

Gly Leu Thr Leu Gly Thr Gly Gly Arg Tyr Thr Gly Ser Ser Tyr Gly
        515                 520                 525

Asp Pro Ala Asn Ser Phe Lys Val Gly Ser Tyr Thr Val Val Asp Ala
    530                 535                 540

Leu Val Arg Tyr Asp Leu Ala Arg Val Gly Met Ala Gly Ser Asn Val
545                 550                 555                 560

Ala Leu His Val Asn Asn Leu Phe Asp Arg Glu Tyr Val Ala Ser Cys
                565                 570                 575

Phe Asn Thr Tyr Gly Cys Phe Trp Gly Ala Glu Arg Gln Val Val Ala
            580                 585                 590

Thr Ala Thr Phe Arg Phe
        595

<210>  5
<211>  1782
<212>  DNA
<213>  artificial

<220>
<223>  artificial

<400>  5
atgaaactga agaacacctt aggcgttgtc atcggctcgc tggttgccgc ttcggcaatg      60

aacgccttcg ccctgaaaga agttcagttt aaagccggta ctgacagcct gttccagact     120

ggttttgact ttagcgattc gttggatgat gacggtgttt actcttatcg cctgaccggt     180

cttgcgcgtt ctgccaatgc ccagcagaaa gggtcagaag agcagcgtta tgctattgca     240

ccggcgttca cctggcgtcc ggatgataaa accaatttta ccttcctttc ttacttccag     300

aacgagccgg aaaccggtta ttacggctgg ttgccgaaag agggaaccgt tgagccgctg     360

ccgaacggta agcgtctgcc gacagacttt aatgaagggg cgaagaacaa cacctattct     420

cgtaatgaga agatggtcgg ctacagcttc gatcacgaat ttaacgacac ctttactgtg 480

cgtcagaacc tgcgctttgc tgaaaacaaa acctcgcaaa acagcgttta tggttacggc 540

gtctgctccg atccggcgaa tgcttacagc aaacagtgtg cggcattagc gccagcggat 600

aaaggccatt atctggcacg taaatacgtc gttgatgatg agaagctgca aaacttctcc 660

gttgataccc agttgcagag caagtttgcc actggcgata tcgaccacac cctgctgacc 720

ggtgtcgact ttatgcgtat gcgtaatgac atcaacgcct ggtttggtta cgacgactct 780

gtgccactgc tcaatctgta caatccggtg aataccgatt tcgacttcaa tgccaaagat 840

ccggcaaact ccggccctta ccgcattctg aataaacaga aacaaacggg cgtttatgtt 900

caggatcagg cgcagtggga taaagtgctg gtcaccctag gcggtcgtta tgactgggca 960

gatcaagaat ctcttaaccg cgttgccggg acgaccgata aacgtgatga caaacagttt 1020

acctggcgtg gtggtgttaa ctacctgttt gataatggtg taacacctta cttcagctat 1080

agcgaatcgt ttttcccttc ttcgcaagtt gggaaggaaa acctgtactt ccagggggat 1140

ggtaatattt tcgcaccgtc taaaggtaag cagtatgaag tcggcgtgaa atatgtaccg 1200

gaagatcgtc cgattgtagt tactggtgcc gtgtataatc tcacttgcac caacgttctg 1260

atggcggacc ctgagggtga gaatctgtac ttccaagggt ccttcttctc ggttgaaggt 1320

ggcgagatcc gcgcacgtgg cgtagaaatc gaagcgaaag cggcgctgtc ggcgagtgtt 1380

aacgtagtcg gttcttatac ttacaccgat gcggaataca ccaccgatac tacctataaa 1440

ggcaatacgc ctgcacaggt gccaaaacac atggcttcgt tgtgggctga ctacaccttc 1500

tttgacggtc cgctttcagg tctgacgctg ggcaccggtg gtcgttatac tggctccagt 1560

tatggtgatc cggctaactc ctttaaagtg ggaagttata cggtcgtgga tgcgttagta 1620

cgttatgatc tggcgcgagt cggcatggct ggctccaacg tggcgctgca tgttaacaac 1680

ctgttcgatc gtgaatacgt cgccagctgc tttaacactt atggctgctt ctggggcgca 1740

gaacgtcagg tcgttgcaac cgcaaccttc cgtttctaat aa 1782

<210> 6
<211> 592
<212> PRT
<213> artificial

<220>
<223> artificial

<400> 6

Met Lys Leu Lys Asn Thr Leu Gly Val Val Ile Gly Ser Leu Val Ala
1               5                   10                  15

Ala Ser Ala Met Asn Ala Phe Ala Leu Lys Glu Val Gln Phe Lys Ala
            20                  25                  30

Gly Thr Asp Ser Leu Phe Gln Thr Gly Phe Asp Phe Ser Asp Ser Leu
        35                  40                  45

Asp Asp Asp Gly Val Tyr Ser Tyr Arg Leu Thr Gly Leu Ala Arg Ser
    50                  55                  60

Ala Asn Ala Gln Gln Lys Gly Ser Glu Glu Gln Arg Tyr Ala Ile Ala
65                  70                  75                  80

Pro Ala Phe Thr Trp Arg Pro Asp Asp Lys Thr Asn Phe Thr Phe Leu
                85                  90                  95

Ser Tyr Phe Gln Asn Glu Pro Glu Thr Gly Tyr Tyr Gly Trp Leu Pro
            100                 105                 110

Lys Glu Gly Thr Val Glu Pro Leu Pro Asn Gly Lys Arg Leu Pro Thr
        115                 120                 125

Asp Phe Asn Glu Gly Ala Lys Asn Asn Thr Tyr Ser Arg Asn Glu Lys
    130                 135                 140

Met Val Gly Tyr Ser Phe Asp His Glu Phe Asn Asp Thr Phe Thr Val
145                 150                 155                 160

Arg Gln Asn Leu Arg Phe Ala Glu Asn Lys Thr Ser Gln Asn Ser Val
                165                 170                 175

Tyr Gly Tyr Gly Val Cys Ser Asp Pro Ala Asn Ala Tyr Ser Lys Gln
            180                 185                 190

Cys Ala Ala Leu Ala Pro Ala Asp Lys Gly His Tyr Leu Ala Arg Lys
        195                 200                 205

Tyr Val Val Asp Asp Glu Lys Leu Gln Asn Phe Ser Val Asp Thr Gln
    210                 215                 220

Leu Gln Ser Lys Phe Ala Thr Gly Asp Ile Asp His Thr Leu Leu Thr
225                 230                 235                 240

Gly Val Asp Phe Met Arg Met Arg Asn Asp Ile Asn Ala Trp Phe Gly
                245                 250                 255

Tyr Asp Asp Ser Val Pro Leu Leu Asn Leu Tyr Asn Pro Val Asn Thr
            260                 265                 270

Asp Phe Asp Phe Asn Ala Lys Asp Pro Ala Asn Ser Gly Pro Tyr Arg

275                 280                 285

Ile Leu Asn Lys Gln Lys Gln Thr Gly Val Tyr Val Gln Asp Gln Ala
    290                 295                 300

Gln Trp Asp Lys Val Leu Val Thr Leu Gly Gly Arg Tyr Asp Trp Ala
305                 310                 315                 320

Asp Gln Glu Ser Leu Asn Arg Val Ala Gly Thr Thr Asp Lys Arg Asp
                325                 330                 335

Asp Lys Gln Phe Thr Trp Arg Gly Gly Val Asn Tyr Leu Phe Asp Asn
            340                 345                 350

Gly Val Thr Pro Tyr Phe Ser Tyr Ser Glu Ser Phe Phe Pro Ser Ser
        355                 360                 365

Gln Val Gly Lys Glu Asn Leu Tyr Phe Gln Gly Asp Gly Asn Ile Phe
    370                 375                 380

Ala Pro Ser Lys Gly Lys Gln Tyr Glu Val Gly Val Lys Tyr Val Pro
385                 390                 395                 400

Glu Asp Arg Pro Ile Val Val Thr Gly Ala Val Tyr Asn Leu Thr Cys
                405                 410                 415

Thr Asn Val Leu Met Ala Asp Pro Glu Gly Glu Asn Leu Tyr Phe Gln
            420                 425                 430

Gly Ser Phe Phe Ser Val Glu Gly Gly Glu Ile Arg Ala Arg Gly Val
        435                 440                 445

Glu Ile Glu Ala Lys Ala Ala Leu Ser Ala Ser Val Asn Val Val Gly
    450                 455                 460

Ser Tyr Thr Tyr Thr Asp Ala Glu Tyr Thr Thr Asp Thr Thr Tyr Lys
465                 470                 475                 480

Gly Asn Thr Pro Ala Gln Val Pro Lys His Met Ala Ser Leu Trp Ala
                485                 490                 495

Asp Tyr Thr Phe Phe Asp Gly Pro Leu Ser Gly Leu Thr Leu Gly Thr
            500                 505                 510

Gly Gly Arg Tyr Thr Gly Ser Ser Tyr Gly Asp Pro Ala Asn Ser Phe
        515                 520                 525

Lys Val Gly Ser Tyr Thr Val Val Asp Ala Leu Val Arg Tyr Asp Leu
    530                 535                 540

Ala Arg Val Gly Met Ala Gly Ser Asn Val Ala Leu His Val Asn Asn
545                 550                 555                 560

Leu Phe Asp Arg Glu Tyr Val Ala Ser Cys Phe Asn Thr Tyr Gly Cys
                565                 570                 575

Phe Trp Gly Ala Glu Arg Gln Val Val Ala Thr Ala Thr Phe Arg Phe
            580                 585                 590

<210>  7
<211>  1773
<212>  DNA
<213>  artificial

<220>
<223>  artificial

<400>  7
atgaaactga agaacacctt aggcgttgtc atcggctcgc tggttgccgc ttcggcaatg      60

aacgccttcg ccctgaaaga agttcagttt aaagccggta ctgacagcct gttccagact     120

ggttttgact ttagcgattc gttggatgat gacggtgttt actcttatcg cctgaccggt     180

cttgcgcgtt ctgccaatgc ccagcagaaa gggtcagaag agcagcgtta tgctattgca     240

ccggcgttca cctggcgtcc ggatgataaa accaatttta ccttcctttc ttacttccag     300

aacgagccgg aaaccggtta ttacggctgg ttgccgaaag agggaaccgt tgagccgctg     360

ccgaacggta agcgtctgcc gacagacttt aatgaagggg cgaagaacaa cacctattct     420

cgtaatgaga agatggtcgg ctacagcttc gatcacgaat ttaacgacac ctttactgtg     480

cgtcagaacc tgcgctttgc tgaaaacaaa acctcgcaaa acagcgttta tggttacggc     540

gtctgctccg atccggcgaa tgcttacagc aaacagtgtg cggcattagc gccagcggat     600

aaaggccatt atctggcacg taaatacgtc gttgatgatg agaagctgca aaacttctcc     660

gttgataccc agttgcagag caagtttgcc actggcgata tcgaccacac cctgctgacc     720

ggtgtcgact ttatgcgtat gcgtaatgac atcaacgcct ggtttggtta cgacgactct     780

gtgccactgc tcaatctgta caatccgtct tctcaccacc accaccacca cggttcttct     840

gtgaataccg atttcgactt caatgccaaa gatccggcaa actccggccc ttaccgcatt     900

ctgaataaac agaaacaaac gggcgtttat gttcaggatc aggcgcagtg ggataaagtg     960

ctggtcaccc taggcggtcg ttatgactgg gcagatcaag aatctcttaa ccgcgttgcc    1020

gggacgaccg ataaacgtga tgacaaacag tttacctggc gtggtggtgt taactacctg    1080

tttgataatg gtgtaacacc ttacttcagc tatagcgaat cgtttgaacc ttcttcgcaa    1140

gttgggaagg atggtaatat tttcgcaccg tctaaaggta agcagtatga agtcggcgtg 1200

aaatatgtac cggaagatcg tccgattgta gttactggtg ccgtgtataa tctcactaaa 1260

accaacaacc tgatggcgga ccctgagggt tccttcttct cggttgaagg tggcgagatc 1320

cgcgcacgtg gcgtagaaat cgaagcgaaa gcggcgctgt cggcgagtgt taacgtagtc 1380

ggttcttata cttacaccga tgcggaatac accaccgata ctacctataa aggcaatacg 1440

cctgcacagg tgccaaaaca catggcttcg ttgtgggctg actacacctt ctttgacggt 1500

ccgctttcag gtctgacgct gggcaccggt ggtcgttata ctggctccag ttatggtgat 1560

ccggctaact cctttaaagt gggaagttat acggtcgtgg atgcgttagt acgttatgat 1620

ctggcgcgag tcggcatggc tggctccaac gtggcgctgc atgttaacaa cctgttcgat 1680

cgtgaatacg tcgccagctg ctttaacact tatggctgct tctggggcgc agaacgtcag 1740

gtcgttgcaa ccgcaacctt ccgtttctaa taa 1773

<210> 8
<211> 589
<212> PRT
<213> artificial

<220>
<223> artificial

<400> 8

Met Lys Leu Lys Asn Thr Leu Gly Val Val Ile Gly Ser Leu Val Ala
1               5                   10                  15

Ala Ser Ala Met Asn Ala Phe Ala Leu Lys Glu Val Gln Phe Lys Ala
            20                  25                  30

Gly Thr Asp Ser Leu Phe Gln Thr Gly Phe Asp Phe Ser Asp Ser Leu
        35                  40                  45

Asp Asp Asp Gly Val Tyr Ser Tyr Arg Leu Thr Gly Leu Ala Arg Ser
    50                  55                  60

Ala Asn Ala Gln Gln Lys Gly Ser Glu Glu Gln Arg Tyr Ala Ile Ala
65                  70                  75                  80

Pro Ala Phe Thr Trp Arg Pro Asp Asp Lys Thr Asn Phe Thr Phe Leu
                85                  90                  95

Ser Tyr Phe Gln Asn Glu Pro Glu Thr Gly Tyr Tyr Gly Trp Leu Pro
            100                 105                 110

Lys Glu Gly Thr Val Glu Pro Leu Pro Asn Gly Lys Arg Leu Pro Thr
        115                 120                 125

Asp Phe Asn Glu Gly Ala Lys Asn Asn Thr Tyr Ser Arg Asn Glu Lys
    130                 135                 140

Met Val Gly Tyr Ser Phe Asp His Glu Phe Asn Asp Thr Phe Thr Val
145                 150                 155                 160

Arg Gln Asn Leu Arg Phe Ala Glu Asn Lys Thr Ser Gln Asn Ser Val
                165                 170                 175

Tyr Gly Tyr Gly Val Cys Ser Asp Pro Ala Asn Ala Tyr Ser Lys Gln
            180                 185                 190

Cys Ala Ala Leu Ala Pro Ala Asp Lys Gly His Tyr Leu Ala Arg Lys
        195                 200                 205

Tyr Val Val Asp Asp Glu Lys Leu Gln Asn Phe Ser Val Asp Thr Gln
    210                 215                 220

Leu Gln Ser Lys Phe Ala Thr Gly Asp Ile Asp His Thr Leu Leu Thr
225                 230                 235                 240

Gly Val Asp Phe Met Arg Met Arg Asn Asp Ile Asn Ala Trp Phe Gly
                245                 250                 255

Tyr Asp Asp Ser Val Pro Leu Leu Asn Leu Tyr Asn Pro Ser Ser His
            260                 265                 270

His His His His His Gly Ser Ser Val Asn Thr Asp Phe Asp Phe Asn
        275                 280                 285

Ala Lys Asp Pro Ala Asn Ser Gly Pro Tyr Arg Ile Leu Asn Lys Gln
    290                 295                 300

Lys Gln Thr Gly Val Tyr Val Gln Asp Gln Ala Gln Trp Asp Lys Val
305                 310                 315                 320

Leu Val Thr Leu Gly Gly Arg Tyr Asp Trp Ala Asp Gln Glu Ser Leu
                325                 330                 335

Asn Arg Val Ala Gly Thr Thr Asp Lys Arg Asp Asp Lys Gln Phe Thr
            340                 345                 350

Trp Arg Gly Gly Val Asn Tyr Leu Phe Asp Asn Gly Val Thr Pro Tyr
        355                 360                 365

Phe Ser Tyr Ser Glu Ser Phe Glu Pro Ser Ser Gln Val Gly Lys Asp

370                 375                 380

Gly Asn Ile Phe Ala Pro Ser Lys Gly Lys Gln Tyr Glu Val Gly Val
385                 390                 395                 400

Lys Tyr Val Pro Glu Asp Arg Pro Ile Val Val Thr Gly Ala Val Tyr
                405                 410                 415

Asn Leu Thr Lys Thr Asn Asn Leu Met Ala Asp Pro Glu Gly Ser Phe
            420                 425                 430

Phe Ser Val Glu Gly Gly Glu Ile Arg Ala Arg Gly Val Glu Ile Glu
        435                 440                 445

Ala Lys Ala Ala Leu Ser Ala Ser Val Asn Val Val Gly Ser Tyr Thr
    450                 455                 460

Tyr Thr Asp Ala Glu Tyr Thr Thr Asp Thr Thr Tyr Lys Gly Asn Thr
465                 470                 475                 480

Pro Ala Gln Val Pro Lys His Met Ala Ser Leu Trp Ala Asp Tyr Thr
                485                 490                 495

Phe Phe Asp Gly Pro Leu Ser Gly Leu Thr Leu Gly Thr Gly Gly Arg
            500                 505                 510

Tyr Thr Gly Ser Ser Tyr Gly Asp Pro Ala Asn Ser Phe Lys Val Gly
        515                 520                 525

Ser Tyr Thr Val Val Asp Ala Leu Val Arg Tyr Asp Leu Ala Arg Val
    530                 535                 540

Gly Met Ala Gly Ser Asn Val Ala Leu His Val Asn Asn Leu Phe Asp
545                 550                 555                 560

Arg Glu Tyr Val Ala Ser Cys Phe Asn Thr Tyr Gly Cys Phe Trp Gly
                565                 570                 575

Ala Glu Arg Gln Val Val Ala Thr Ala Thr Phe Arg Phe
            580                 585

<210>  9
<211>  2193
<212>  DNA
<213>  artificial

<220>
<223>  artificial

<400> 9
atgaaacaga gcaccattgc gctggcgctg ctgccgctgc tgtttacccc ggtgaccaaa 60
gcggctagct gcgccattaa actggttcag agcccgaatg gtaattttgc agcaagcttt 120
gttctggatg gcaccaaatg gatcttcaaa agcaaatact atgacagcag caaaggttat 180
tgggtgggta tttatgaagt gtgggatcgc aaagctgcag ccgcagcagc agctgccgca 240
gccggcagcg aaaacctgta cttccagggg agcggcggcg cgccggtgcc gtatccggat 300
ccgctggaac cgcgcgcggc gagcgcgccg agcccgtata gcaccctggt ggtgtttggc 360
gatgcgctga gcgatgcggg ccagtttccg gatccggcgg gcccggcggg cagcaccagc 420
cgctttacca accgcgtggg cccgacctat cagaacggca gcggcgaaat ttttggcccg 480
accgcgccga tgctgctggg caaccagctg ggcattgcgc cgggcgatct ggcggcgagc 540
accagcccgg tgaacgcgca gcagggcatt gcggatggca acaactgggc ggtgggcggc 600
tatcgcaccg atcagattta tgatagcatt accgcggcga acggcagcct gattgaacgc 660
gataacaccc tgctgcgcag ccgcgatggc tatctggtgg atcgcgcgcg ccagggcctg 720
ggcgcggatc cgaacgcgct gtattatatt accggcggcg gcaacgattt tctgcagggc 780
cgcattctga acgatgtgca ggcgcagcag gcggcgggcc gcctggtgga tagcgtgcag 840
gcgctgcagc aggcgggcgc gcgctatatt gtggtgtggc tgctgccgga tctgggcctg 900
accccggcga cctttggcgg cccgctgcag ccgtttgcga gccagctgag cggcaccttt 960
aacgcggaac tgaccgcgca gctgagccag gcgggcgcga acgtgattcc gctgaacatt 1020
ccgctgctgc tgaaagaagg catggcgaac ccggcgagct ttggcctggc ggcggatcag 1080
aacctgattg gcacctgctt tagcggcaac ggctgcacca tgaacccgac ctatggcatt 1140
aacggcagca ccccggatcc gagcaaactg ctgtttaacg atagcgtgca tccgaccatt 1200
accggccagc gcctgattgc ggattatacc tatagcctgc tgagcgcgcc gtgggaactg 1260
accctgctgc cggaaatggc gcatggcacc ctgcgcgcgt atcaggatga actgcgcagc 1320
cagtggcagg cggattggga aaactggcag aacgtgggcc agtggcgcgg ctttgtgggc 1380
ggcggcggcc agcgcctgga ttttgatagc caggatagcg cggcgagcgg cgatggcaac 1440
ggctataacc tgaccctggg cggcagctat cgcattgatg aagcgtggcg cgcgggcgtg 1500
gcggcgggct tttatcgcca gaaactggaa gcgggcgcga aagatagcga ttatcgcatg 1560
aacagctata tggcgagcgc gtttgtgcag tatcaggaaa accgctggtg ggcggatgcg 1620
gcgctgaccg gcggctatct ggattatgat gatctgaaac gcaaatttgc gctgggcggc 1680
ggcgaacgca gcgaaaaagg cgataccaac ggccatctgt gggcgtttag cgcgcgcctg 1740
ggctatgata ttgcgcagca ggcggatagc ccgtggcatc tgagcccgtt tgtgagcgcg 1800
gattatgcgc gcgtggaagt ggatggctat agcgaaaaag gcgcgagcgc gaccgcgctg 1860

gattatgatg atcagaaacg cagcagcaaa cgcctgggcg cgggcctgca gggcaaatat     1920

gcgtttggca gcgataccca gctgtttgcg gaatatgcgc atgaacgcga atatgaagat     1980

gatacccagg atctgaccat gagcctgaac agcctgccgg gcaaccgctt taccctggaa     2040

ggctataccc cgcaggatca tctgaaccgc gtgagcctgg gctttagcca gaaactggcg     2100

ccggaactga gcctgcgcgg cggctataac tggcgcaaag gcgaagatga tacccagcag     2160

agcgtgagcc tggcgctgag cctggatttt taa                                  2193

<210>  10
<211>  730
<212>  PRT
<213>  artificial

<220>
<223>  artificial

<400>  10

Met Lys Gln Ser Thr Ile Ala Leu Ala Leu Leu Pro Leu Leu Phe Thr
1               5                   10                  15

Pro Val Thr Lys Ala Ala Ser Cys Ala Ile Lys Leu Val Gln Ser Pro
            20                  25                  30

Asn Gly Asn Phe Ala Ala Ser Phe Val Leu Asp Gly Thr Lys Trp Ile
        35                  40                  45

Phe Lys Ser Lys Tyr Tyr Asp Ser Ser Lys Gly Tyr Trp Val Gly Ile
    50                  55                  60

Tyr Glu Val Trp Asp Arg Lys Ala Ala Ala Ala Ala Ala Ala Ala Ala
65                  70                  75                  80

Ala Gly Ser Glu Asn Leu Tyr Phe Gln Gly Ser Gly Gly Ala Pro Val
                85                  90                  95

Pro Tyr Pro Asp Pro Leu Glu Pro Arg Ala Ala Ser Ala Pro Ser Pro
            100                 105                 110

Tyr Ser Thr Leu Val Val Phe Gly Asp Ala Leu Ser Asp Ala Gly Gln
        115                 120                 125

Phe Pro Asp Pro Ala Gly Pro Ala Gly Ser Thr Ser Arg Phe Thr Asn
    130                 135                 140

Arg Val Gly Pro Thr Tyr Gln Asn Gly Ser Gly Glu Ile Phe Gly Pro
145                 150                 155                 160

Thr Ala Pro Met Leu Leu Gly Asn Gln Leu Gly Ile Ala Pro Gly Asp
                165                 170                 175

Leu Ala Ala Ser Thr Ser Pro Val Asn Ala Gln Gln Gly Ile Ala Asp
            180                 185                 190

Gly Asn Asn Trp Ala Val Gly Gly Tyr Arg Thr Asp Gln Ile Tyr Asp
        195                 200                 205

Ser Ile Thr Ala Ala Asn Gly Ser Leu Ile Glu Arg Asp Asn Thr Leu
    210                 215                 220

Leu Arg Ser Arg Asp Gly Tyr Leu Val Asp Arg Ala Arg Gln Gly Leu
225                 230                 235                 240

Gly Ala Asp Pro Asn Ala Leu Tyr Tyr Ile Thr Gly Gly Gly Asn Asp
                245                 250                 255

Phe Leu Gln Gly Arg Ile Leu Asn Asp Val Gln Ala Gln Gln Ala Ala
            260                 265                 270

Gly Arg Leu Val Asp Ser Val Gln Ala Leu Gln Gln Ala Gly Ala Arg
        275                 280                 285

Tyr Ile Val Val Trp Leu Leu Pro Asp Leu Gly Leu Thr Pro Ala Thr
    290                 295                 300

Phe Gly Gly Pro Leu Gln Pro Phe Ala Ser Gln Leu Ser Gly Thr Phe
305                 310                 315                 320

Asn Ala Glu Leu Thr Ala Gln Leu Ser Gln Ala Gly Ala Asn Val Ile
                325                 330                 335

Pro Leu Asn Ile Pro Leu Leu Leu Lys Glu Gly Met Ala Asn Pro Ala
            340                 345                 350

Ser Phe Gly Leu Ala Ala Asp Gln Asn Leu Ile Gly Thr Cys Phe Ser
        355                 360                 365

Gly Asn Gly Cys Thr Met Asn Pro Thr Tyr Gly Ile Asn Gly Ser Thr
    370                 375                 380

Pro Asp Pro Ser Lys Leu Leu Phe Asn Asp Ser Val His Pro Thr Ile
385                 390                 395                 400

Thr Gly Gln Arg Leu Ile Ala Asp Tyr Thr Tyr Ser Leu Leu Ser Ala
                405                 410                 415

Pro Trp Glu Leu Thr Leu Leu Pro Glu Met Ala His Gly Thr Leu Arg
        420                 425                 430

Ala Tyr Gln Asp Glu Leu Arg Ser Gln Trp Gln Ala Asp Trp Glu Asn
    435                 440                 445

Trp Gln Asn Val Gly Gln Trp Arg Gly Phe Val Gly Gly Gly Gly Gln
    450                 455                 460

Arg Leu Asp Phe Asp Ser Gln Asp Ser Ala Ala Ser Gly Asp Gly Asn
465                 470                 475                 480

Gly Tyr Asn Leu Thr Leu Gly Gly Ser Tyr Arg Ile Asp Glu Ala Trp
                485                 490                 495

Arg Ala Gly Val Ala Ala Gly Phe Tyr Arg Gln Lys Leu Glu Ala Gly
        500                 505                 510

Ala Lys Asp Ser Asp Tyr Arg Met Asn Ser Tyr Met Ala Ser Ala Phe
    515                 520                 525

Val Gln Tyr Gln Glu Asn Arg Trp Trp Ala Asp Ala Ala Leu Thr Gly
    530                 535                 540

Gly Tyr Leu Asp Tyr Asp Asp Leu Lys Arg Lys Phe Ala Leu Gly Gly
545                 550                 555                 560

Gly Glu Arg Ser Glu Lys Gly Asp Thr Asn Gly His Leu Trp Ala Phe
                565                 570                 575

Ser Ala Arg Leu Gly Tyr Asp Ile Ala Gln Gln Ala Asp Ser Pro Trp
        580                 585                 590

His Leu Ser Pro Phe Val Ser Ala Asp Tyr Ala Arg Val Glu Val Asp
    595                 600                 605

Gly Tyr Ser Glu Lys Gly Ala Ser Ala Thr Ala Leu Asp Tyr Asp Asp
    610                 615                 620

Gln Lys Arg Ser Ser Lys Arg Leu Gly Ala Gly Leu Gln Gly Lys Tyr
625                 630                 635                 640

Ala Phe Gly Ser Asp Thr Gln Leu Phe Ala Glu Tyr Ala His Glu Arg
                645                 650                 655

Glu Tyr Glu Asp Asp Thr Gln Asp Leu Thr Met Ser Leu Asn Ser Leu
        660                 665                 670

Pro Gly Asn Arg Phe Thr Leu Glu Gly Tyr Thr Pro Gln Asp His Leu
        675                 680                 685

Asn Arg Val Ser Leu Gly Phe Ser Gln Lys Leu Ala Pro Glu Leu Ser
    690                 695                 700

Leu Arg Gly Gly Tyr Asn Trp Arg Lys Gly Glu Asp Asp Thr Gln Gln
705                 710                 715                 720

Ser Val Ser Leu Ala Leu Ser Leu Asp Phe
                725                 730

<210>  11
<211>  2190
<212>  DNA
<213>  artificial

<220>
<223>  artificial

<400>  11
atgaaacaga gcaccattgc gctggcgctg ctgccgctgc tgtttacccc ggtgaccaaa      60

gcggctagcg ccattaaact ggttcagagc ccgaatggta attttgcagc aagctttgtt     120

ctggatggca ccaaatggat cttcaaaagc aaatactatg acagcagcaa aggttattgg     180

gtgggtattt atgaagtgtg ggatcgcaaa gctgcagccg cagcagcagc tgccgcagcc     240

ggcagcgaaa acctgtactt ccaggggagc ggcggcgcgc cggtgccgta tccggatccg     300

ctggaaccgc gcgcggcgag cgcgccgagc ccgtatagca ccctggtggt gtttggcgat     360

gcgctgagcg atgcgggcca gtttccggat ccggcgggcc cggcgggcag caccagccgc     420

tttaccaacc gcgtgggccc gacctatcag aacggcagcg gcgaaatttt tggcccgacc     480

gcgccgatgc tgctgggcaa ccagctgggc attgcgccgg gcgatctggc ggcgagcacc     540

agcccggtga acgcgcagca gggcattgcg gatggcaaca actgggcggt gggcggctat     600

cgcaccgatc agatttatga tagcattacc gcggcgaacg gcagcctgat tgaacgcgat     660

aacaccctgc tgcgcagccg cgatggctat ctggtggatc gcgcgcgcca gggcctgggc     720

gcggatccga acgcgctgta ttatattacc ggcggcggca acgattttct gcagggccgc     780

attctgaacg atgtgcaggc gcagcaggcg gcgggccgcc tggtggatag cgtgcaggcg     840

ctgcagcagg cgggcgcgcg ctatattgtg gtgtggctgc tgccggatct gggcctgacc     900

ccggcgacct ttggcggccc gctgcagccg tttgcgagcc agctgagcgg cacctttaac     960

gcggaactga ccgcgcagct gagccaggcg ggcgcgaacg tgattccgct gaacattccg    1020

ctgctgctga aagaaggcat ggcgaacccg gcgagctttg gcctggcggc ggatcagaac    1080

ctgattggca cctgctttag cggcaacggc tgcaccatga acccgaccta tggcattaac 1140

ggcagcaccc cggatccgag caaactgctg tttaacgata gcgtgcatcc gaccattacc 1200

ggccagcgcc tgattgcgga ttatacctat agcctgctga gcgcgccgtg ggaactgacc 1260

ctgctgccgg aaatggcgca tggcaccctg cgcgcgtatc aggatgaact gcgcagccag 1320

tggcaggcgg attgggaaaa ctggcagaac gtgggccagt ggcgcggctt tgtgggcggc 1380

ggcggccagc gcctggattt tgatagccag gatagcgcgg cgagcggcga tggcaacggc 1440

tataacctga ccctgggcgg cagctatcgc attgatgaag cgtggcgcgc gggcgtggcg 1500

gcgggctttt atcgccagaa actggaagcg ggcgcgaaag atagcgatta tcgcatgaac 1560

agctatatgg cgagcgcgtt tgtgcagtat caggaaaacc gctggtgggc ggatgcggcg 1620

ctgaccggcg gctatctgga ttatgatgat ctgaaacgca aatttgcgct gggcggcggc 1680

gaacgcagcg aaaaaggcga taccaacggc catctgtggg cgtttagcgc gcgcctgggc 1740

tatgatattg cgcagcaggc ggatagcccg tggcatctga gcccgtttgt gagcgcggat 1800

tatgcgcgcg tggaagtgga tggctatagc gaaaaaggcg cgagcgcgac cgcgctggat 1860

tatgatgatc agaaacgcag cagcaaacgc ctgggcgcgg gcctgcaggg caaatatgcg 1920

tttggcagcg atacccagct gtttgcggaa tatgcgcatg aacgcgaata tgaagatgat 1980

acccaggatc tgaccatgag cctgaacagc ctgccgggca accgctttac cctggaaggc 2040

tataccccgc aggatcatct gaaccgcgtg agcctgggct ttagccagaa actggcgccg 2100

gaactgagcc tgcgcggcgg ctataactgg cgcaaaggcg aagatgatac ccagcagagc 2160

gtgagcctgg cgctgagcct ggatttttaa 2190

<210> 12
<211> 729
<212> PRT
<213> artificial

<220>
<223> artificial

<400> 12

Met Lys Gln Ser Thr Ile Ala Leu Ala Leu Leu Pro Leu Leu Phe Thr
1               5                   10                  15

Pro Val Thr Lys Ala Ala Ser Ala Ile Lys Leu Val Gln Ser Pro Asn
            20                  25                  30

Gly Asn Phe Ala Ala Ser Phe Val Leu Asp Gly Thr Lys Trp Ile Phe
        35                  40                  45

Lys Ser Lys Tyr Tyr Asp Ser Ser Lys Gly Tyr Trp Val Gly Ile Tyr
    50                  55                  60

Glu Val Trp Asp Arg Lys Ala Ala Ala Ala Ala Ala Ala Ala Ala Ala
65                  70                  75                  80

Gly Ser Glu Asn Leu Tyr Phe Gln Gly Ser Gly Gly Ala Pro Val Pro
                85                  90                  95

Tyr Pro Asp Pro Leu Glu Pro Arg Ala Ala Ser Ala Pro Ser Pro Tyr
            100                 105                 110

Ser Thr Leu Val Val Phe Gly Asp Ala Leu Ser Asp Ala Gly Gln Phe
        115                 120                 125

Pro Asp Pro Ala Gly Pro Ala Gly Ser Thr Ser Arg Phe Thr Asn Arg
    130                 135                 140

Val Gly Pro Thr Tyr Gln Asn Gly Ser Gly Glu Ile Phe Gly Pro Thr
145                 150                 155                 160

Ala Pro Met Leu Leu Gly Asn Gln Leu Gly Ile Ala Pro Gly Asp Leu
                165                 170                 175

Ala Ala Ser Thr Ser Pro Val Asn Ala Gln Gln Gly Ile Ala Asp Gly
            180                 185                 190

Asn Asn Trp Ala Val Gly Gly Tyr Arg Thr Asp Gln Ile Tyr Asp Ser
        195                 200                 205

Ile Thr Ala Ala Asn Gly Ser Leu Ile Glu Arg Asp Asn Thr Leu Leu
    210                 215                 220

Arg Ser Arg Asp Gly Tyr Leu Val Asp Arg Ala Arg Gln Gly Leu Gly
225                 230                 235                 240

Ala Asp Pro Asn Ala Leu Tyr Tyr Ile Thr Gly Gly Gly Asn Asp Phe
                245                 250                 255

Leu Gln Gly Arg Ile Leu Asn Asp Val Gln Ala Gln Gln Ala Ala Gly
            260                 265                 270

Arg Leu Val Asp Ser Val Gln Ala Leu Gln Gln Ala Gly Ala Arg Tyr
        275                 280                 285

Ile Val Val Trp Leu Leu Pro Asp Leu Gly Leu Thr Pro Ala Thr Phe
    290                 295                 300

Gly Gly Pro Leu Gln Pro Phe Ala Ser Gln Leu Ser Gly Thr Phe Asn

305                 310                 315                 320

Ala Glu Leu Thr Ala Gln Leu Ser Gln Ala Gly Ala Asn Val Ile Pro
                325                 330                 335

Leu Asn Ile Pro Leu Leu Leu Lys Glu Gly Met Ala Asn Pro Ala Ser
            340                 345                 350

Phe Gly Leu Ala Ala Asp Gln Asn Leu Ile Gly Thr Cys Phe Ser Gly
        355                 360                 365

Asn Gly Cys Thr Met Asn Pro Thr Tyr Gly Ile Asn Gly Ser Thr Pro
    370                 375                 380

Asp Pro Ser Lys Leu Leu Phe Asn Asp Ser Val His Pro Thr Ile Thr
385                 390                 395                 400

Gly Gln Arg Leu Ile Ala Asp Tyr Thr Tyr Ser Leu Leu Ser Ala Pro
                405                 410                 415

Trp Glu Leu Thr Leu Leu Pro Glu Met Ala His Gly Thr Leu Arg Ala
            420                 425                 430

Tyr Gln Asp Glu Leu Arg Ser Gln Trp Gln Ala Asp Trp Glu Asn Trp
        435                 440                 445

Gln Asn Val Gly Gln Trp Arg Gly Phe Val Gly Gly Gly Gly Gln Arg
    450                 455                 460

Leu Asp Phe Asp Ser Gln Asp Ser Ala Ala Ser Gly Asp Gly Asn Gly
465                 470                 475                 480

Tyr Asn Leu Thr Leu Gly Gly Ser Tyr Arg Ile Asp Glu Ala Trp Arg
                485                 490                 495

Ala Gly Val Ala Ala Gly Phe Tyr Arg Gln Lys Leu Glu Ala Gly Ala
            500                 505                 510

Lys Asp Ser Asp Tyr Arg Met Asn Ser Tyr Met Ala Ser Ala Phe Val
        515                 520                 525

Gln Tyr Gln Glu Asn Arg Trp Trp Ala Asp Ala Ala Leu Thr Gly Gly
    530                 535                 540

Tyr Leu Asp Tyr Asp Asp Leu Lys Arg Lys Phe Ala Leu Gly Gly Gly
545                 550                 555                 560

Glu Arg Ser Glu Lys Gly Asp Thr Asn Gly His Leu Trp Ala Phe Ser
                565                 570                 575

Ala Arg Leu Gly Tyr Asp Ile Ala Gln Gln Ala Asp Ser Pro Trp His
            580                 585                 590

Leu Ser Pro Phe Val Ser Ala Asp Tyr Ala Arg Val Glu Val Asp Gly
        595                 600                 605

Tyr Ser Glu Lys Gly Ala Ser Ala Thr Ala Leu Asp Tyr Asp Asp Gln
    610                 615                 620

Lys Arg Ser Ser Lys Arg Leu Gly Ala Gly Leu Gln Gly Lys Tyr Ala
625                 630                 635                 640

Phe Gly Ser Asp Thr Gln Leu Phe Ala Glu Tyr Ala His Glu Arg Glu
                645                 650                 655

Tyr Glu Asp Asp Thr Gln Asp Leu Thr Met Ser Leu Asn Ser Leu Pro
            660                 665                 670

Gly Asn Arg Phe Thr Leu Glu Gly Tyr Thr Pro Gln Asp His Leu Asn
        675                 680                 685

Arg Val Ser Leu Gly Phe Ser Gln Lys Leu Ala Pro Glu Leu Ser Leu
    690                 695                 700

Arg Gly Gly Tyr Asn Trp Arg Lys Gly Glu Asp Asp Thr Gln Gln Ser
705                 710                 715                 720

Val Ser Leu Ala Leu Ser Leu Asp Phe
                725

<210>  13
<211>  2514
<212>  DNA
<213>  artificial

<220>
<223>  artificial

<400>  13
atgaaacaga gcaccattgc gctggcgctg ctgccgctgc tgtttacccc ggtgaccaaa      60

gcggctagca atcaactgca acaactgcaa aatccgggcg agagtccgcc ggttcatccg     120

ttcgtggcac cgctgtccta tctgctgggt acctggcgcg gccagggtga aggcgagtat     180

ccgaccattc cgagctttcg ctatggcgaa gagatccgtt tcagccattc gggtaaaccg     240

gtgattgcct atacccaaaa aacgtggaaa ctggaatcgg gtgcaccgct gctggcagag     300

agtggttatt ttcgcccgcg tccggatggt tctattgaag tggttatcgc atgctcgacc 360

ggtctggtgg aagttcaaaa aggcacgtat aatgtggatg agcagagtat taaactgaaa 420

tctgacctgg tgggcaacgc gtccaaagtt aaagaaatca gccgcgaatt cgagctggtt 480

gacggtaaac tgagttatgt ggttcgtctg agcacgacca cgaatccgct gcaaccgctg 540

ctgaaagcca tcctggacaa actgggcagc gaaaacctgt acttccaggg gagcggcggc 600

gcgccggtgc cgtatccgga tccgctggaa ccgcgcgcgg cgagcgcgcc gagcccgtat 660

agcaccctgg tggtgtttgg cgatgcgctg agcgatgcgg gccagtttcc ggatccggcg 720

ggcccggcgg gcagcaccag ccgctttacc aaccgcgtgg gcccgaccta tcagaacggc 780

agcggcgaaa tttttggccc gaccgcgccg atgctgctgg gcaaccagct gggcattgcg 840

ccgggcgatc tggcggcgag caccagcccg gtgaacgcgc agcagggcat tgcggatggc 900

aacaactggg cggtgggcgg ctatcgcacc gatcagattt atgatagcat taccgcggcg 960

aacggcagcc tgattgaacg cgataacacc ctgctgcgca gccgcgatgg ctatctggtg 1020

gatcgcgcgc gccagggcct gggcgcggat ccgaacgcgc tgtattatat taccggcggc 1080

ggcaacgatt ttctgcaggg ccgcattctg aacgatgtgc aggcgcagca ggcggcgggc 1140

cgcctggtgg atagcgtgca ggcgctgcag caggcgggcg cgcgctatat tgtggtgtgg 1200

ctgctgccgg atctgggcct gaccccggcg acctttggcg gcccgctgca gccgtttgcg 1260

agccagctga gcggcacctt taacgcggaa ctgaccgcgc agctgagcca ggcgggcgcg 1320

aacgtgattc cgctgaacat tccgctgctg ctgaaagaag gcatggcgaa cccggcgagc 1380

tttggcctgg cggcggatca gaacctgatt ggcacctgct ttagcggcaa cggctgcacc 1440

atgaacccga cctatggcat taacggcagc accccggatc cgagcaaact gctgtttaac 1500

gatagcgtgc atccgaccat taccggccag cgcctgattg cggattatac ctatagcctg 1560

ctgagcgcgc cgtgggaact gaccctgctg ccggaaatgg cgcatggcac cctgcgcgcg 1620

tatcaggatg aactgcgcag ccagtggcag gcggattggg aaaactggca gaacgtgggc 1680

cagtggcgcg gctttgtggg cggcggcggc cagcgcctgg attttgatag ccaggatagc 1740

gcggcgagcg gcgatggcaa cggctataac ctgaccctgg gcggcagcta tcgcattgat 1800

gaagcgtggc gcgcgggcgt ggcggcgggc ttttatcgcc agaaactgga agcgggcgcg 1860

aaagatagcg attatcgcat gaacagctat atggcgagcg cgtttgtgca gtatcaggaa 1920

aaccgctggt gggcggatgc ggcgctgacc ggcggctatc tggattatga tgatctgaaa 1980

cgcaaatttg cgctgggcgg cggcgaacgc agcgaaaaag gcgataccaa cggccatctg 2040

tgggcgttta gcgcgcgcct gggctatgat attgcgcagc aggcggatag cccgtggcat 2100

ctgagcccgt ttgtgagcgc ggattatgcg cgcgtggaag tggatggcta tagcgaaaaa 2160

ggcgcgagcg cgaccgcgct ggattatgat gatcagaaac gcagcagcaa acgcctgggc 2220

gcgggcctgc agggcaaata tgcgtttggc agcgataccc agctgtttgc ggaatatgcg 2280

catgaacgcg aatatgaaga tgatacccag gatctgacca tgagcctgaa cagcctgccg 2340

ggcaaccgct ttaccctgga aggctatacc ccgcaggatc atctgaaccg cgtgagcctg 2400

ggctttagcc agaaactggc gccggaactg agcctgcgcg gcggctataa ctggcgcaaa 2460

ggcgaagatg atacccagca gagcgtgagc ctggcgctga gcctggattt ttaa 2514

<210> 14
<211> 837
<212> PRT
<213> artificial

<220>
<223> artificial

<400> 14

Met Lys Gln Ser Thr Ile Ala Leu Ala Leu Leu Pro Leu Leu Phe Thr
1               5                   10                  15

Pro Val Thr Lys Ala Ala Ser Asn Gln Leu Gln Gln Leu Gln Asn Pro
            20                  25                  30

Gly Glu Ser Pro Pro Val His Pro Phe Val Ala Pro Leu Ser Tyr Leu
        35                  40                  45

Leu Gly Thr Trp Arg Gly Gln Gly Glu Gly Glu Tyr Pro Thr Ile Pro
    50                  55                  60

Ser Phe Arg Tyr Gly Glu Glu Ile Arg Phe Ser His Ser Gly Lys Pro
65                  70                  75                  80

Val Ile Ala Tyr Thr Gln Lys Thr Trp Lys Leu Glu Ser Gly Ala Pro
                85                  90                  95

Leu Leu Ala Glu Ser Gly Tyr Phe Arg Pro Arg Pro Asp Gly Ser Ile
            100                 105                 110

Glu Val Val Ile Ala Cys Ser Thr Gly Leu Val Glu Val Gln Lys Gly
        115                 120                 125

Thr Tyr Asn Val Asp Glu Gln Ser Ile Lys Leu Lys Ser Asp Leu Val
    130                 135                 140

Gly Asn Ala Ser Lys Val Lys Glu Ile Ser Arg Glu Phe Glu Leu Val
145                 150                 155                 160

Asp Gly Lys Leu Ser Tyr Val Val Arg Leu Ser Thr Thr Thr Asn Pro

                165                 170                 175

Leu Gln Pro Leu Leu Lys Ala Ile Leu Asp Lys Leu Gly Ser Glu Asn
            180                 185                 190

Leu Tyr Phe Gln Gly Ser Gly Gly Ala Pro Val Pro Tyr Pro Asp Pro
        195                 200                 205

Leu Glu Pro Arg Ala Ala Ser Ala Pro Ser Pro Tyr Ser Thr Leu Val
    210                 215                 220

Val Phe Gly Asp Ala Leu Ser Asp Ala Gly Gln Phe Pro Asp Pro Ala
225                 230                 235                 240

Gly Pro Ala Gly Ser Thr Ser Arg Phe Thr Asn Arg Val Gly Pro Thr
                245                 250                 255

Tyr Gln Asn Gly Ser Gly Glu Ile Phe Gly Pro Thr Ala Pro Met Leu
            260                 265                 270

Leu Gly Asn Gln Leu Gly Ile Ala Pro Gly Asp Leu Ala Ala Ser Thr
        275                 280                 285

Ser Pro Val Asn Ala Gln Gln Gly Ile Ala Asp Gly Asn Asn Trp Ala
    290                 295                 300

Val Gly Gly Tyr Arg Thr Asp Gln Ile Tyr Asp Ser Ile Thr Ala Ala
305                 310                 315                 320

Asn Gly Ser Leu Ile Glu Arg Asp Asn Thr Leu Leu Arg Ser Arg Asp
                325                 330                 335

Gly Tyr Leu Val Asp Arg Ala Arg Gln Gly Leu Gly Ala Asp Pro Asn
            340                 345                 350

Ala Leu Tyr Tyr Ile Thr Gly Gly Gly Asn Asp Phe Leu Gln Gly Arg
        355                 360                 365

Ile Leu Asn Asp Val Gln Ala Gln Gln Ala Ala Gly Arg Leu Val Asp
    370                 375                 380

Ser Val Gln Ala Leu Gln Gln Ala Gly Ala Arg Tyr Ile Val Val Trp
385                 390                 395                 400

Leu Leu Pro Asp Leu Gly Leu Thr Pro Ala Thr Phe Gly Gly Pro Leu
                405                 410                 415

Gln Pro Phe Ala Ser Gln Leu Ser Gly Thr Phe Asn Ala Glu Leu Thr
        420                 425                 430

Ala Gln Leu Ser Gln Ala Gly Ala Asn Val Ile Pro Leu Asn Ile Pro
    435                 440                 445

Leu Leu Leu Lys Glu Gly Met Ala Asn Pro Ala Ser Phe Gly Leu Ala
    450                 455                 460

Ala Asp Gln Asn Leu Ile Gly Thr Cys Phe Ser Gly Asn Gly Cys Thr
465                 470                 475                 480

Met Asn Pro Thr Tyr Gly Ile Asn Gly Ser Thr Pro Asp Pro Ser Lys
                485                 490                 495

Leu Leu Phe Asn Asp Ser Val His Pro Thr Ile Thr Gly Gln Arg Leu
            500                 505                 510

Ile Ala Asp Tyr Thr Tyr Ser Leu Leu Ser Ala Pro Trp Glu Leu Thr
        515                 520                 525

Leu Leu Pro Glu Met Ala His Gly Thr Leu Arg Ala Tyr Gln Asp Glu
    530                 535                 540

Leu Arg Ser Gln Trp Gln Ala Asp Trp Glu Asn Trp Gln Asn Val Gly
545                 550                 555                 560

Gln Trp Arg Gly Phe Val Gly Gly Gly Gly Gln Arg Leu Asp Phe Asp
                565                 570                 575

Ser Gln Asp Ser Ala Ala Ser Gly Asp Gly Asn Gly Tyr Asn Leu Thr
            580                 585                 590

Leu Gly Gly Ser Tyr Arg Ile Asp Glu Ala Trp Arg Ala Gly Val Ala
        595                 600                 605

Ala Gly Phe Tyr Arg Gln Lys Leu Glu Ala Gly Ala Lys Asp Ser Asp
    610                 615                 620

Tyr Arg Met Asn Ser Tyr Met Ala Ser Ala Phe Val Gln Tyr Gln Glu
625                 630                 635                 640

Asn Arg Trp Trp Ala Asp Ala Ala Leu Thr Gly Gly Tyr Leu Asp Tyr
                645                 650                 655

Asp Asp Leu Lys Arg Lys Phe Ala Leu Gly Gly Gly Glu Arg Ser Glu
            660                 665                 670

Lys Gly Asp Thr Asn Gly His Leu Trp Ala Phe Ser Ala Arg Leu Gly
        675                 680                 685

Tyr Asp Ile Ala Gln Gln Ala Asp Ser Pro Trp His Leu Ser Pro Phe
    690                 695                 700

Val Ser Ala Asp Tyr Ala Arg Val Glu Val Asp Gly Tyr Ser Glu Lys
705                 710                 715                 720

Gly Ala Ser Ala Thr Ala Leu Asp Tyr Asp Asp Gln Lys Arg Ser Ser
                725                 730                 735

Lys Arg Leu Gly Ala Gly Leu Gln Gly Lys Tyr Ala Phe Gly Ser Asp
            740                 745                 750

Thr Gln Leu Phe Ala Glu Tyr Ala His Glu Arg Glu Tyr Glu Asp Asp
        755                 760                 765

Thr Gln Asp Leu Thr Met Ser Leu Asn Ser Leu Pro Gly Asn Arg Phe
    770                 775                 780

Thr Leu Glu Gly Tyr Thr Pro Gln Asp His Leu Asn Arg Val Ser Leu
785                 790                 795                 800

Gly Phe Ser Gln Lys Leu Ala Pro Glu Leu Ser Leu Arg Gly Gly Tyr
                805                 810                 815

Asn Trp Arg Lys Gly Glu Asp Asp Thr Gln Gln Ser Val Ser Leu Ala
            820                 825                 830

Leu Ser Leu Asp Phe
        835

<210> 15
<211> 2514
<212> DNA
<213> artificial

<220>
<223> artificial

<400> 15
atgaaacaga gcaccattgc gctggcgctg ctgccgctgc tgtttacccc ggtgaccaaa 60

gcggctagca atcaactgca acaactgcaa aatccgggcg agagtccgcc ggttcatccg 120

ttcgtggcac cgctgtccta tctgctgggt acctggcgcg gccagggtga aggcgagtat 180

ccgaccattc cgagctttcg ctatggcgaa gagatccgtt tcagccattc gggtaaaccg 240

gtgattgcct atacccaaaa aacgtggaaa ctggaatcgg gtgcaccgct gctggcagag 300

agtggttatt ttcgcccgcg tccggatggt tctattgaag tggttatcgc acagtcgacc 360

ggtctggtgg aagttcaaaa aggcacgtat aatgtggatg agcagagtat taaactgaaa 420

tctgacctgg tgggcaacgc gtccaaagtt aaagaaatca gccgcgaatt cgagctggtt 480

gacggtaaac tgagttatgt ggttcgtctg agcacgacca cgaatccgct gcaaccgctg 540

ctgaaagcca tcctggacaa actgggcagc gaaaacctgt acttccaggg gagcggcggc 600

gcgccggtgc cgtatccgga tccgctggaa ccgcgcgcgg cgagcgcgcc gagcccgtat 660

agcaccctgg tggtgtttgg cgatgcgctg agcgatgcgg gccagtttcc ggatccggcg 720

ggcccggcgg gcagcaccag ccgctttacc aaccgcgtgg gcccgaccta tcagaacggc 780

agcggcgaaa tttttggccc gaccgcgccg atgctgctgg gcaaccagct gggcattgcg 840

ccgggcgatc tggcggcgag caccagcccg gtgaacgcgc agcagggcat tgcggatggc 900

aacaactggg cggtgggcgg ctatcgcacc gatcagattt atgatagcat taccgcggcg 960

aacggcagcc tgattgaacg cgataacacc ctgctgcgca gccgcgatgg ctatctggtg 1020

gatcgcgcgc gccagggcct gggcgcggat ccgaacgcgc tgtattatat taccggcggc 1080

ggcaacgatt ttctgcaggg ccgcattctg aacgatgtgc aggcgcagca ggcggcgggc 1140

cgcctggtgg atagcgtgca ggcgctgcag caggcgggcg cgcgctatat tgtggtgtgg 1200

ctgctgccgg atctgggcct gaccccggcg acctttggcg gcccgctgca gccgtttgcg 1260

agccagctga gcggcacctt taacgcggaa ctgaccgcgc agctgagcca ggcgggcgcg 1320

aacgtgattc cgctgaacat tccgctgctg ctgaaagaag gcatggcgaa cccggcgagc 1380

tttggcctgg cggcggatca gaacctgatt ggcacctgct ttagcggcaa cggctgcacc 1440

atgaacccga cctatggcat taacggcagc accccggatc cgagcaaact gctgtttaac 1500

gatagcgtgc atccgaccat taccggccag cgcctgattg cggattatac ctatagcctg 1560

ctgagcgcgc cgtgggaact gaccctgctg ccggaaatgg cgcatggcac cctgcgcgcg 1620

tatcaggatg aactgcgcag ccagtggcag gcggattggg aaaactggca gaacgtgggc 1680

cagtggcgcg gctttgtggg cggcggcggc cagcgcctgg attttgatag ccaggatagc 1740

gcggcgagcg gcgatggcaa cggctataac ctgaccctgg gcggcagcta tcgcattgat 1800

gaagcgtggc gcgcgggcgt ggcggcgggc ttttatcgcc agaaactgga agcgggcgcg 1860

aaagatagcg attatcgcat gaacagctat atggcgagcg cgtttgtgca gtatcaggaa 1920

aaccgctggt gggcggatgc ggcgctgacc ggcggctatc tggattatga tgatctgaaa 1980

cgcaaatttg cgctgggcgg cggcgaacgc agcgaaaaag gcgataccaa cggccatctg 2040

tgggcgttta gcgcgcgcct gggctatgat attgcgcagc aggcggatag cccgtggcat 2100

ctgagcccgt ttgtgagcgc ggattatgcg cgcgtggaag tggatggcta tagcgaaaaa 2160

ggcgcgagcg cgaccgcgct ggattatgat gatcagaaac gcagcagcaa acgcctgggc 2220

gcgggcctgc agggcaaata tgcgtttggc agcgataccc agctgtttgc ggaatatgcg 2280

catgaacgcg aatatgaaga tgatacccag gatctgacca tgagcctgaa cagcctgccg 2340

ggcaaccgct ttaccctgga aggctatacc ccgcaggatc atctgaaccg cgtgagcctg 2400

ggctttagcc agaaactggc gccggaactg agcctgcgcg gcggctataa ctggcgcaaa 2460

ggcgaagatg atacccagca gagcgtgagc ctggcgctga gcctggattt ttaa 2514

<210> 16
<211> 837
<212> PRT
<213> artificial

<220>
<223> artificial

<400> 16

Met Lys Gln Ser Thr Ile Ala Leu Ala Leu Leu Pro Leu Leu Phe Thr
1               5                   10                  15

Pro Val Thr Lys Ala Ala Ser Asn Gln Leu Gln Gln Leu Gln Asn Pro
            20                  25                  30

Gly Glu Ser Pro Pro Val His Pro Phe Val Ala Pro Leu Ser Tyr Leu
        35                  40                  45

Leu Gly Thr Trp Arg Gly Gln Gly Glu Gly Glu Tyr Pro Thr Ile Pro
    50                  55                  60

Ser Phe Arg Tyr Gly Glu Glu Ile Arg Phe Ser His Ser Gly Lys Pro
65                  70                  75                  80

Val Ile Ala Tyr Thr Gln Lys Thr Trp Lys Leu Glu Ser Gly Ala Pro
                85                  90                  95

Leu Leu Ala Glu Ser Gly Tyr Phe Arg Pro Arg Pro Asp Gly Ser Ile
            100                 105                 110

Glu Val Val Ile Ala Gln Ser Thr Gly Leu Val Glu Val Gln Lys Gly
        115                 120                 125

Thr Tyr Asn Val Asp Glu Gln Ser Ile Lys Leu Lys Ser Asp Leu Val
    130                 135                 140

Gly Asn Ala Ser Lys Val Lys Glu Ile Ser Arg Glu Phe Glu Leu Val
145                 150                 155                 160

Asp Gly Lys Leu Ser Tyr Val Val Arg Leu Ser Thr Thr Thr Asn Pro
                165                 170                 175

Leu Gln Pro Leu Leu Lys Ala Ile Leu Asp Lys Leu Gly Ser Glu Asn
            180                 185                 190

Leu Tyr Phe Gln Gly Ser Gly Gly Ala Pro Val Pro Tyr Pro Asp Pro
        195                 200                 205

Leu Glu Pro Arg Ala Ala Ser Ala Pro Ser Pro Tyr Ser Thr Leu Val
    210                 215                 220

Val Phe Gly Asp Ala Leu Ser Asp Ala Gly Gln Phe Pro Asp Pro Ala
225                 230                 235                 240

Gly Pro Ala Gly Ser Thr Ser Arg Phe Thr Asn Arg Val Gly Pro Thr
                245                 250                 255

Tyr Gln Asn Gly Ser Gly Glu Ile Phe Gly Pro Thr Ala Pro Met Leu
            260                 265                 270

Leu Gly Asn Gln Leu Gly Ile Ala Pro Gly Asp Leu Ala Ala Ser Thr
        275                 280                 285

Ser Pro Val Asn Ala Gln Gln Gly Ile Ala Asp Gly Asn Asn Trp Ala
    290                 295                 300

Val Gly Gly Tyr Arg Thr Asp Gln Ile Tyr Asp Ser Ile Thr Ala Ala
305                 310                 315                 320

Asn Gly Ser Leu Ile Glu Arg Asp Asn Thr Leu Leu Arg Ser Arg Asp
                325                 330                 335

Gly Tyr Leu Val Asp Arg Ala Arg Gln Gly Leu Gly Ala Asp Pro Asn
            340                 345                 350

Ala Leu Tyr Tyr Ile Thr Gly Gly Gly Asn Asp Phe Leu Gln Gly Arg
        355                 360                 365

Ile Leu Asn Asp Val Gln Ala Gln Gln Ala Ala Gly Arg Leu Val Asp
    370                 375                 380

Ser Val Gln Ala Leu Gln Gln Ala Gly Ala Arg Tyr Ile Val Val Trp
385                 390                 395                 400

Leu Leu Pro Asp Leu Gly Leu Thr Pro Ala Thr Phe Gly Gly Pro Leu
                405                 410                 415

Gln Pro Phe Ala Ser Gln Leu Ser Gly Thr Phe Asn Ala Glu Leu Thr
        420                 425                 430

Ala Gln Leu Ser Gln Ala Gly Ala Asn Val Ile Pro Leu Asn Ile Pro
    435                 440                 445

Leu Leu Leu Lys Glu Gly Met Ala Asn Pro Ala Ser Phe Gly Leu Ala
    450                 455                 460

Ala Asp Gln Asn Leu Ile Gly Thr Cys Phe Ser Gly Asn Gly Cys Thr
465                 470                 475                 480

Met Asn Pro Thr Tyr Gly Ile Asn Gly Ser Thr Pro Asp Pro Ser Lys
                485                 490                 495

Leu Leu Phe Asn Asp Ser Val His Pro Thr Ile Thr Gly Gln Arg Leu
            500                 505                 510

Ile Ala Asp Tyr Thr Tyr Ser Leu Leu Ser Ala Pro Trp Glu Leu Thr
        515                 520                 525

Leu Leu Pro Glu Met Ala His Gly Thr Leu Arg Ala Tyr Gln Asp Glu
    530                 535                 540

Leu Arg Ser Gln Trp Gln Ala Asp Trp Glu Asn Trp Gln Asn Val Gly
545                 550                 555                 560

Gln Trp Arg Gly Phe Val Gly Gly Gly Gly Gln Arg Leu Asp Phe Asp
                565                 570                 575

Ser Gln Asp Ser Ala Ala Ser Gly Asp Gly Asn Gly Tyr Asn Leu Thr
            580                 585                 590

Leu Gly Gly Ser Tyr Arg Ile Asp Glu Ala Trp Arg Ala Gly Val Ala
        595                 600                 605

Ala Gly Phe Tyr Arg Gln Lys Leu Glu Ala Gly Ala Lys Asp Ser Asp
    610                 615                 620

Tyr Arg Met Asn Ser Tyr Met Ala Ser Ala Phe Val Gln Tyr Gln Glu
625                 630                 635                 640

Asn Arg Trp Trp Ala Asp Ala Ala Leu Thr Gly Gly Tyr Leu Asp Tyr
                645                 650                 655

Asp Asp Leu Lys Arg Lys Phe Ala Leu Gly Gly Gly Glu Arg Ser Glu
            660                 665                 670

Lys Gly Asp Thr Asn Gly His Leu Trp Ala Phe Ser Ala Arg Leu Gly
        675                 680                 685

Tyr Asp Ile Ala Gln Gln Ala Asp Ser Pro Trp His Leu Ser Pro Phe
    690                 695                 700

Val Ser Ala Asp Tyr Ala Arg Val Glu Val Asp Gly Tyr Ser Glu Lys
705                 710                 715                 720

Gly Ala Ser Ala Thr Ala Leu Asp Tyr Asp Asp Gln Lys Arg Ser Ser
                725                 730                 735

Lys Arg Leu Gly Ala Gly Leu Gln Gly Lys Tyr Ala Phe Gly Ser Asp
            740                 745                 750

Thr Gln Leu Phe Ala Glu Tyr Ala His Glu Arg Glu Tyr Glu Asp Asp
        755                 760                 765

Thr Gln Asp Leu Thr Met Ser Leu Asn Ser Leu Pro Gly Asn Arg Phe
    770                 775                 780

Thr Leu Glu Gly Tyr Thr Pro Gln Asp His Leu Asn Arg Val Ser Leu
785                 790                 795                 800

Gly Phe Ser Gln Lys Leu Ala Pro Glu Leu Ser Leu Arg Gly Gly Tyr
                805                 810                 815

Asn Trp Arg Lys Gly Glu Asp Asp Thr Gln Gln Ser Val Ser Leu Ala
            820                 825                 830

Leu Ser Leu Asp Phe
        835

<210>  17
<211>  24
<212>  PRT
<213>  artificial

<220>
<223>  artificial

<400>  17

Met Lys Leu Lys Asn Thr Leu Gly Val Val Ile Gly Ser Leu Val Ala
1               5                   10                  15

Ala Ser Ala Met Asn Ala Phe Ala
            20

<210>  18
<211>  72
<212>  DNA
<213>  artificial

<220>
<223>  artificial

<400>  18
atgaaactga agaacacctt aggcgttgtc atcggctcgc tggttgccgc ttcggcaatg      60

aacgccttcg cc                                                          72

## Beschreibung

Die vorliegende Erfindung betrifft gemäß einem primären Aspekt ein Biohybridkatalysator-System, umfassend oder bestehend aus (a) einer intakten, nicht-menschlichen Zelle und (b) einem oder mehreren künstlichen Metalloenzym(en) bestehend aus (b1) einem Proteingerüst und (b2) einem an das Proteingerüst angebundenen, katalytisch aktiven Metallkomplex (wie nachfolgend definiert), wobei das/die Metalloenzym(e) in der Zellmembran verankert und/oder an der Zelloberfläche der Zelle angebunden ist/sind.

Gemäß weiteren Aspekten betrifft die vorliegende Erfindung ein Verfahren zur Herstellung des erfindungsgemäßen Biohybridkatalysator-Systems, ein Verfahren zur Katalyse einer chemischen Reaktion und die Verwendung des erfindungsgemäßen Biohybridkatalysator-Systems als Katalysator zur Katalyse einer chemischen Reaktion.

Weitere Aspekte der vorliegenden Erfindung ergeben sich aus der nachfolgenden Beschreibung, insbesondere den Beispielen, sowie den beigefügten Patentansprüchen.

Biohybridkatalysatoren stellen eine Klasse von Katalysatoren dar, die aus Übergangsmetallkomplexen gebunden an Biomoleküle, beispielsweise Proteine, bestehen. Biokatalytische Umsetzungen werden dabei verbunden mit umgebungskontrollierten Reaktionen hoher katalytischer Aktivität und Selektivität und milden Reaktionsbedingungen. Übergangsmetallkatalyse hingegen zeichnet sich durch Kontrolle mittels physikalischer Parameter aus und zeigt hohe Vielfalt an verwendbaren Lösungsmitteln und Metallen für verschiedenste Reaktionen. Die Verbindung der komplementären Eigenschaften der Protein-Liganden-Sphäre mit hochaktiven Metallkomplexen ermöglicht daher die Bereitstellung bio-anorganischer Katalysatoren, die Metallkomplexe mit höherer Selektivität ausstatten, Reaktionen beschleunigen und systematische Verbesserungen erlauben.

Biohybridkatalysatoren ermöglichen durch diese einzigartige Kombination erfolgreicher Prinzipien der Biokatalyse mit denen der homogegen chemischen Katalyse eine Vielzahl neuer selektiver Reaktionen. Sie stellen eine eigene Katalysatorklasse dar, die in der Natur nicht biokatalytisch verwendete Metalle mit der spezifischen Umgebung von Proteinen (Substratorientierung durch 20 Bausteine in Form von Aminosäuren spezifisch im Raum) für Selektivität zugänglich machen kann. Letzteres ist unerreicht mit chemischer Synthese trotz verschiedener Ansätze in der supramolekularen Katalyse.

Das in der chemischen Katalyse eingesetzte, synthetisch oft herausfordernde Finetuning der vergleichsweise kleinen Metall-assoziierten Liganden erlaubt nur eine eingeschränkte Kontrolle von Substratorientierungen. Bisher verlässt man sich für die Optimierung von Übergangsmetallkatalysatoren für Aktivität, Spezifität und Selektivität so gut wie ausschließlich auf das Finetuning der Metall-assoziierten Liganden der ersten Ligandensphäre. Die Vorgehensweise zur Verbesserung der Katalysatoreigenschaften besteht dabei im Wesentlichen aus der Identifikation aktiver Metallkomplexe, dem Einführen und Testen kleiner Veränderungen (z.B. von -OH-Gruppen; sterischer Abschirmung etc.) sowie dem Überprüfen verwandter Strukturen. Kleine Liganden ermöglichen jedoch keine spezifische Substratbindung und können daher keine umfassende sterische Kontrolle der Reaktion gewährleisten.

Die Anbindung von Metallkomplexen an Proteine kann durch dative oder supramolekulare Wechselwirkungen erfolgen, sowie durch kovalente Modifizierung von und Anbindung durch Aminosäureseitenketten. Besonders Letzteres erlaubt eine definierte Bindung und Orientierung der Metallkomplexe innerhalb der Proteinumgebung auch bei umfassenden Änderungen der Umgebung mittels Protein Engineering.

Beim derzeitigen Stand der Technik ist der industrielle Einsatz der Biohybridkatalysatoren bisher eingeschränkt durch zu hohe Kosten bei der Synthese, vor allem auf Grund der nötigen Reinigung der Proteine vor der Bindung der katalytischen Komplexe. Der Einsatzbereich der Biohybridkatalysatoren ist zudem meist auf ihre natürliche Stabilitätsumgebung beschränkt, so dass die Verwendung organischer Lösungsmittel, extremer pH-Werte und hoher Temperaturen sowie nicht wasserlöslicher Substrate dem Reaktionsspektrum enge Grenzen setzt. Das Potential von Protein Engineering zur Verbesserung von Stabilität, Aktivität und Selektivität wird mit den bisher verfügbaren isolierten Proteinstrukturen nicht ausgenutzt, da keine Hochdurchsatzdurchmusterungsmethoden verfügbar sind. Die bisher eingesetzten Methoden zur Generierung von Diversität beschränken sich daher auf einzelne Positionsmutationen (SDM) oder Einzelpositionssaturierungen (SSM) basierend auf bekannten Kristallstrukturen.

Bisher durchgeführte Protein Engineering-Ansätze mit Biohybridkatalysatoren in ganzen Zellen beschränken sich derzeit zudem auf lösliche Proteine im Cytoplasma (Wilson, Y. M.; Dürrenberger, M.; Nogueira, E. S.; Ward, T. R., JACS, 2014, 136 (25), 8928-8932; Dürrenberger, M.; Heinisch, T.; Wilson, Y. M.; Rossel, T.; Nogueira, E.; Knörr, L.; Mutschler, A.; Kersten, K.; Zimbron, M. J.; Pierron, J.; Schirmer, T.; Ward, T. R, Angewandte Chemie (International Ed. in English) 2011, 50 (13), 3026-3029.) oder die Expression von Biohybridkatalysatoren im Periplasma von *E. coli* (M. Jeschek et al., Nature 2016, 537, 661-665). Dieser Ansatz ist jedoch limitiert durch die begrenzten Aufnahmefähigkeiten von Metallkomplexen und Substraten durch die Zellen. Zusätzlich ist die überwiegende Anzahl der Übergangsmetallkomplexe sehr empfindlich gegenüber nukleophilen Angriffen durch Zellplasmabestandteile (wie z.B. Glutathion), was zum Verlust der Aktivität des Katalysators führt.

Primäre Aufgabe der vorliegenden Erfindung war es daher, Biohybridkatalysator-Systeme bereitzustellen, die die oben genannten Probleme des Standes der Technik überwinden, insbesondere die Bereitstellung von kostengünstigen Biohybridkatalysator-Systemen, die die Umsetzung auch von nicht-wasserlöslichen bzw. nur zum Teil wasserlöslichen Substraten unter Verwendung organischer Lösungsmittel, extremeren pH-Werten und höheren Temperaturen als in natürlicher (Protein-)Reaktionsumgebung ermöglichen.

Zudem war es eine Aufgabe der vorliegenden Erfindung, Biohybridkatalysator-Systeme bereitzustellen, die dem Protein Engineering zur Verbesserung von Stabilität, Aktivität und Selektivität der Systeme zugänglich sind. Im Protein Engineering können durch Gelenkte Evolution mittels Simultansaturierungsmethoden (OmniChange) bis zu 3,2 Millionen Varianten pro Tag generiert werden. Die bisher entwickelten Biohybridkatalysatoren (basierend auf Streptavidin und Serumalbuminen) sind das Produkt umfassender Optimierungen, jedoch wurden Protein Engineering-Konzepte bisher nicht konsequent verfolgt. Die Hauptlimitierung ist dabei die Gewinnung ausreichender Biohybridkatalysatormengen für Synthese, Reinigung, Charakterisierung und Katalyse.

Überraschenderweise wurde in, im Rahmen der vorliegenden Erfindung durchgeführten, Studien nun herausgefunden, dass der Einsatz von Biohybridkatalysatoren in Ganzzellsystemen die Verwendung von State-of-the-art-Methoden der Gelenkten Evolution erlaubt, die es möglich macht, die Proteinumgebung des Übergangskatalysators maßzuschneidern. Dies ermöglicht es, die Stabilität, Selektivität und Aktivität des Biohybridkatalysators stark zu verbessern und das Synthesepotential von in der Natur nicht eingesetzten Metallen voll auszuschöpfen.

Analog zu den bereits überwiegend eingesetzten Ganzzell-Katalysatoren im industriellen Maßstab (z.B. in der Synthese von Acrylamid aus Acrylnitril) erlauben die hierin beschriebenen Systeme (im Gegensatz zu isolierten Biohybridkatalysatoren) zudem kostengünstige Verfahren in Lösung. Die ganzen Zellen ermöglichen des Weiteren den Schutz der Proteinstruktur vor denaturierenden Bedingungen (z.B. durch Änderungen im pH-Wert, der Umgebungstemperatur oder die Präsenz organischer Lösungsmittel) und benötigen keine vorhergehende Aufreinigung.

Die oben genannten Aufgaben werden daher erfindungsgemäß gelöst durch ein Biohybridkatalysator-System, umfassend oder bestehend aus
(a) einer intakten, nicht-menschlichen Zelle und
(b) einem oder mehreren künstlichen Metalloenzym(en) bestehend aus
   (b1) einem Proteingerüst und
   (b2) einem an das Proteingerüst angebundenen, katalytisch aktiven Metallkomplex, enthaltend ein Übergangsmetall, vorzugsweise ein Metall ausgewählt aus der Gruppe bestehend aus Ruthenium, Rhodium, Cobalt, Kupfer, Iridium, Mangan, Palladium und Platin,
wobei das/die Metalloenzym(e) in der Zellmembran verankert und/oder an der Zelloberfläche der Zelle angebunden ist/sind und
wobei der Metallkomplex ausgewählt ist aus der Gruppe bestehend aus organometallischen Katalysatoren, vorzugsweise Grubbs-Hoveyda-Katalysatoren, Rh-Cyclopentadien-Katalysatoren, Rh-Carben-Katalysatoren, Cu-Carben-Katalysatoren, Cu-Terpyridin-Katalysatoren, Cu-Phenanthrolin-Katalysatoren, Cu-Pyren-Katalysatoren, Co-Terpyridin-Katalysatoren, Pt-Terpyridin-Katalysatoren, Pt-Carben-Katalysatoren, Pd-Terpyridin-Katalysatoren, Pd-Carben-Katalysatoren, Ir-Carben-Katalysatoren und Mn-Cyclopentadienyl-Katalysatoren.

Gemäß einer bevorzugten Ausführungsform ist/sind das/die künstliche(n) Metalloenzym(e) (b) bzw. ist das Proteingerüst (b1) Bestandteil der intakten, nicht-menschlichen Zelle (a), vorzugsweise Bestandteil der äußeren Zellmembran der intakten, nicht-menschlichen Zelle (a).

Insgesamt ist es bevorzugt, wenn das/die Metalloenzym(e) (b) überwiegend, bevorzugt ausschließlich, zumindest nahezu ausschließlich, in der Zellmembran verankert und/oder an der Zelloberfläche der Zelle angebunden ist/sind - und nicht in der Zelle, insbesondere nicht im Cytosol, vorliegen.

Gemäß einer weiteren bevorzugten Ausführungsform, ist/sind das/die Metalloenzym(e) (b) so in der Zelle verankert bzw. an der Zelle angebunden, dass es/sie auf der Zelloberfläche bzw. an der Zellaußenseite für Reaktionen verfügbar ist/sind bzw. an der Zellaußenseite (vorzugsweise an der Zelloberfläche) katalytisch aktiv sein kann/können.

Gemäß einer bevorzugten Ausführungsform, besitzt das Proteingerüst (b1) für sich allein (d.h. ohne den an das Proteingerüst angebundenen, katalytisch aktiven Metallkomplex (b2)) keine katalytische Aktivität.

Im Rahmen des vorliegenden Textes handelt es sich bei dem katalytisch aktiven Metallkomplex um einen metallorganischen Katalysator (wie vorangehend definiert), in dem (mindestens) ein Metall(ion) wie vorangehend definiert von den oben definierten organischen Liganden komplexiert vorliegt.

Die Anbindung eines solchen katalytisch aktiven Metallkomplexes an ein freies, d.h. nicht in der Zellmembran verankertes und/oder an der Zelloberfläche der Zelle angebundenes, Proteingerüst wird beispielsweise durch die folgende Publikation offenbart: Fukumoto, K., Onoda, A., Mizohata, E., Bocola, M., Inoue, T., Schwaneberg, U., Hayashi, T., Rhodium-Complex-Linked Hybrid Biocatalyst: Stereo-Controlled Phenylacetylene Polymerization within an Engineered Protein Cavity, ChemCatChem 2014, 6, 1229-1235.

In einem System, in dem das/die künstliche(n) Metalloenzym(e) in der Zellmembran verankert und/oder an der Zelloberfläche der Zelle angebunden ist/sind, ist es wahrscheinlich, dass die Faltung des Proteingerüsts beim Einbau in die Zellmembran bzw. bei der Anbindung an die Zelloberfläche verändert wird im Vergleich zur Faltung des freien Proteingerüsts. Eine veränderte Faltung des Proteingerüsts würde eine Anbindung des katalytisch aktiven Metallkomplexes am Proteingerüst bzw. die Substratzugänglichkeit zum angebundenen Metallkomplex negativ beeinflussen, was einen Verlust oder eine Verschlechterung der katalytischen Aktivität des Systems zur Folge hätte. Auch die Anbindung des katalytisch aktiven Metallkomplexes am in der Zellmembran verankerten bzw. an die Zelloberfläche gebundenen Proteingerüst kann eine Veränderung der Faltung des Proteingerüsts bewirken. Zudem kann die Orientierung des in der Zellmembran verankerten bzw. an die Zelloberfläche gebundenen Proteingerüsts unter Umständen die Anbindung des katalytisch aktiven Metallkomplexes am Proteingerüst behindern bzw. die Zugänglichkeit für Substrate des Systems negativ beeinflussen (durch eine geringere Orientierungsmöglichkeit im Vergleich zum freien Protein). Überraschenderweise trat keiner dieser Nachteile im oben beschriebenen, erfindungsgemäßen Biohybridkatalysator-System auf.

In dem vorteilhaften Fall, dass die Verankerung des Proteingerüsts in der Zellmembran bzw. dessen Anbindung an der Zelloberfläche und die Anbindung des katalytisch aktiven Metallkomplexes an das gebundene Proteingerüst keinen Einfluss auf die katalytische Aktivität des Systems und die Zugänglichkeit des Substrats hat, könnte durch die Verankerung / Anbindung dennoch die Selektivität des Systems in chemischen Reaktionen durch Änderung der Proteinumgebung und damit der sterischen Bedingungen um das aktive Metallzentrum verändert werden. In diesem Fall würden stereoselektive Reaktionen (wie weiter unten beschrieben) überhaupt nicht mehr oder mit verschlechterter Ausbeute und/oder verschlechterter Stereoselektivität ablaufen. Auch keiner dieser Nachteile wurde überraschenderweise im erfindungsgemäßen Biohybridkatalysator-System beobachtet.

Es ist des Weiteren bekannt, dass der Einbau von Membranproteinen in Zellmembranen die Zellmembranen leicht durchgängig für verschiedene Substanzen machen kann (von innen nach außen und *vice versa*), wodurch toxische Stoffe aus dem Inneren der Zelle an die Zellmembran bzw. Zelloberfläche gelangen können. Daher lag die Vermutung nahe, dass auch Stoffe aus der Zelle austreten können, die das/die künstliche(n) Metalloenzym(e) negativ beeinflussen können. Überraschenderweise trat jedoch auch dieser Effekt im erfindungsgemäßen Biohybridkatalysator-System nicht auf.

Die Verwendung der erfindungsgemäßen Biohybridkatalysator-Systeme in der industriellen Stoffproduktion (z.B. als Katalysatoren bei der Herstellung von Pharma-Intermediaten) ist besonders vorteilhaft, da sie die Entwicklung kostengünstiger Biohybridkatalyseprozesse ermöglicht.

Biohybridkatalyseprozesse minimieren vorteilhafterweise im Vergleich zur klassischen Übergansmetallkatalyse (z.B. basierend auf Ru, Rh oder Pd-Übergangsmetallkomplexen) die Aufarbeitungskosten und -zeit stark, da die Abtrennung des in ganzen Zellen inkorporierten Katalysators sehr einfach, vorliegend vorzugsweise durch Abzentrifugieren des erfindungsgemäßen Biohybridkatalysator-Systems, erfolgt.

Moderne aktive Pharmazeutika (API) lassen für den Einsatz im Menschen einen maximalen Metallgehalt von 20-200 ppm zu. Die Metallentfernung ist dabei nicht trivial, da neuere pharmazeutische Wirkstoffe meist elektronenpaarreiche, heterozyklische Verbindungen sind, die als starke Metallchelate bekannt sind (Magano, Javier, "Large-Scale Applications of Transition Metal Removal Techniques in the Manufacture of Pharmaceuticals", In Transition Metal-Catalyzed Couplings in Process Chemistry, 313-355, Wiley-VCH, 2013). Bei den erfindungsgemäßen Biohybridkatalysator-Systemen ist eine zusätzliche aufwändige Reinigung des Produktes von Metallrückstanden vorteilhafterweise nicht erforderlich, da die Biohybridkatalysator-Systeme mitsamt ihrer metallischen Katalyse-Komponente (b2) - dem an das Proteingerüst angebundene, katalytisch aktiven Metallkomplex - effizient durch z. B. Zentrifugieren abgetrennt werden können.

Biohybridkatalysatoren ermöglichen das Synthesepotential von in der Natur nicht eingesetzten Übergangsmetallen auszuschöpfen. Durch Lyophillisierung und definierte Rehydrierung der erfindungsgemäßen Biohybridkatalysator-Systeme kann der Einsatz in der industriellen Stoffproduktion vorteilhafterweise nicht nur in Wasser, sondern auch in (organischen) Lösungsmitteln erfolgen.

Die Verankerung des/der künstlichen Metalloenzyms/enzyme (b) in der Zellmembran der intakten, nicht-menschlichen Zelle (a) stabilisiert vorteilhafterweise das/die Metalloenzym(e) und erweitert dadurch die Anwendungsmöglichkeiten und die Reaktionsvielfalt des erfindungsgemäßen Biohybridkatalysator-Systems bis zu reinem Lösungsmittel oder Verfahren in denen direkt das Substrat als Lösungsmittel verwendet wird. Vorteilhafterweise wird/werden durch die besagte Verankerung und Stabilisierung des/der Metalloenzyms/enzyme (b) der Anwendungsbereich des erfindungsgemäßen Biohybridkatalysator-Systems auf höhere Temperaturen und/oder extremere pH-Werte (beispielsweise stärkere Abweichungen von neutralem pH-Wert in den sauren oder basischen pH-Bereich) als solche, die in natürlicher Proteinumgebung - insbesondere innerhalb lebender Zellen - herrschen, erweitert.

Die erfindungsgemäßen Biohybridkatalysator-Systeme mit Zelloberflächenanbindung und/oder Membranexpression und/oder -verankerung des/der Metalloenzyms/enzyme ermöglichen des Weiteren vorteilhafterweise höhere Katalysator-Aktivitäten durch die hohe Zugänglichkeit der aktiven Komponenten an der Zelloberfläche.

Da das/die Metalloenzym(e) (b) in den erfindungsgemäßen Biohybridkatalysator-Systemen so in der Zelle verankert bzw. an der Zelle angebunden bzw. orientiert ist/sind, dass es/sie auf der Zelloberfläche bzw. an der Zellaußenseite für Reaktionen verfügbar ist/sind bzw. an der Zellaußenseite (vorzugsweise an der Zelloberfläche) katalytisch aktiv sein kann/können, liegt vorteilhafterweise keine Diffusionsbarriere durch die äußere Zellmembran, die üblicherweise die Substrataufnahme verhindert oder stark verringert, vor.

Zudem kann in den erfindungsgemäßen Biohybridkatalysator-Systemen eine deutlich höhere Katalysatorbeladung des Proteingerüsts (b1) - und damit des gesamten Biohybridkatalysator-Systems - vorliegen, wenn die vorzugsweise kovalente Verankerung des katalytisch aktiven Metallkomplexes (b2) im Proteingerüst (b1) bei basischem pH-Wert erfolgt.

Das erfindungsgemäße Biohybridkatalysator-System umfassend in der Zellmembran verankerte und/oder an der Zelloberfläche der Zelle angebundene Metalloenzyme (b) ermöglicht vorteilhafterweise unterschiedlichste Bedingungen der unmittelbaren Katalysatorumgebung (z.B. betreffend pH-Wert, Lösemittel oder Temperatur).

Das erfindungsgemäße Biohybridkatalysator-System, bei welchem das/die Metalloenzym(e) (b) bzw. das Proteingerüst (b1) in der Zellmembran verankert und/oder an der Zelloberfläche der Zelle angebunden ist/sind, beschränkt sich vorteilhafterweise nicht auf den Einsatz von besonders säure- und oxidationsbeständigen Proteingerüsten, die z.B. für die geringen pH-Werte im Periplasma notwendig wären.

Die Optimierung der Katalysatoreigenschaften des erfindungsgemäßen Biohybridkatalysator-Systems mittels Protein Engineering ermöglicht vorteilhafterweise das Generieren einer Vielzahl an Biohybridkatalysatorvarianten (>10⁵) und deren effiziente Durchmusterung.

Ein weiterer Vorteil des erfindungsgemäßen Biohybridkatalysator-Systems ist, dass es nach einer katalytischen Reaktion durch, z.B. Zentrifugation, einfach vom Reaktionsgemisch abgetrennt und wiederverwertet werden kann. Die Zellen (a) des Biohybridkatalysator-Systems müssen zudem vorteilhafterweise zur Gewinnung des Katalyseproduktes nicht zerstört werden, da die Produkte an der Zellaußenseite generiert werden. Dadurch kann das Biohybridkatalysator-System nach der Reaktion einfach und effizient vom synthetisierten Produkt abgetrennt und ggf. wiederverwendet werden.

Eine weitere bevorzugte Ausführungsform betrifft ein Biohybridkatalysator-System wie vorangehend definiert, wobei die Zelle ausgewählt ist aus der Gruppe bestehend aus Bakterienzellen, vorzugsweise gram-negativen Bakterienzellen, wie *E. coli* und *Pseudomonas.*

Der Einsatz gram-negativer Bakterienzellen als intakte, nicht-menschliche Zelle des erfindungsgemäßen Biohybridkatalysator-Systems ist besonders vorteilhaft, da diese sehr gut erforschte und etablierte Zellsysteme sind, die durch ihren strukturellen Aufbau zudem besonders gut geeignet für die funktionale Einlagerung (d.h. Verankerung oder Anbindung) der Gerüstproteine an bzw. in die äußere Zellmembran sind.

Unter einer "intakten" Zelle ist im Rahmen der vorliegenden Erfindung eine Zelle zu verstehen, die wenigstens eine intakte Zellmembran und/oder Zellwand aufweist.

Vorzugsweise weist das erfindungsgemäße Biohybridkatalysator-System in der äußeren Zellmembran eine hohe Dichte an künstlichen Metalloenzymen auf, um eine möglichst hohe Katalysatoraktivität des Biohybridkatalysator-Systems zu gewährleisten. Bevorzugt ist eine Oberflächenbeladung von mindestens 10.000 künstlichen Metalloenzymen, vorzugsweise mindestens 20.000, besonders bevorzugt mindestens 30.000 oder gar mindestens 100.000, pro Zelle.

Neben den oben erwähnten gram-negativen Bakterienzellen eigen sich für das erfindungsgemäße Biohybridkatalysator-System prinzipiell ebenso gram-positive Bakterien, wie Bacillus-Stämme, da gram-positive Bakterien mit ihrer Cytoplasmamembran ebenfalls über eine Zellmembran verfügen. Im Rahmen der vorliegenden Erfindung können beispielsweise Corynebakterien als Zellen verwendet werden.

Eine weitere bevorzugte Ausführungsform betrifft ein Biohybridkatalysator-System wie vorangehend definiert, wobei das Proteingerüst ausgewählt ist aus der Gruppe bestehend aus Transmembranproteinen und löslichen Proteinen, vorzugsweise solchen mit beta-Fassstruktur, vorzugsweise solche mit 6 bis 24 beta-Faltblättern, bevorzugt FhuA (Ferric hydroxamate uptake protein component A) oder Nitrobindin, membranständigen oder verankerten Proteinen, vorzugsweise LCI. Das antimikrobielle Peptid LCI wurde erstmals aus *B*. *subtilis* A014 isoliert (Liu J, Pan N, Chen Z., Rice Genet Newsl. 1990;7:151-4).

Der Einsatz von Transmembranproteinen und löslichen Proteinen, vorzugsweise solchen mit beta-Fassstruktur, insbesondere der Einsatz des Transmembranproteins FhuA und des löslichen Proteins Nitrobindin, als Proteingerüst im erfindungsgemäßen Biohybridkatalysator-System ist besonders vorteilhaft, da er durch die großen Kavitäten der Gerüstproteine (FhuA: 20x24 Å, Nitrobindin: 12x14 Å) eine Positionierung der katalytischen Komplexe tief im Inneren der Fassstruktur und nicht nur an der Proteinoberfläche ermöglicht. Die tiefe Positionierung führt zu einer umfassenden Kontrolle der Umgebung des katalytisch wirksamen Metallkomplexes durch spezifisch austauschbare Aminosäuren des Proteingerüsts und damit zur Steuerung der Selektivität des Metallkomplexes.

LCI ist gekennzeichnet durch eine flache beta-Faltblattstruktur bestehend aus 47 Aminosäuren und hat eine Größe von 5460 Da. 23 Wasserstoffbrückenbindungen sorgen für eine stabile Struktur mit hoher Thermoresistenz (Liu J, Li Z, Pan N, Chen Z., Chin J Biotechnol. 1992;8(3):187-93). Nach Anbindung eines Katalysatorkomplexes an ein freies Cystein erlaubt die hydrophobe und flache LCI-Struktur eine einfache systematische Verbesserung der Proteinumgebung mittels Gelenkter Evolution. LCI hat als antimikrobielles Peptid eine starke Affinität zu hydrophoben Oberflächen (wie z.B, Polypropylen oder Polystyrene), was LCI-basierende Beschichtungstechnologien ermöglicht.

Die Lokalisierung des Proteingerüsts (z.B. von FhuA oder Nitrobindin) in der äußeren Zellmembran als Transmembranprotein bzw. als membranverankertes Protein umgeht die Membran als Diffusionsbarriere und hält deaktivierende Nukleophile innerhalb der Zellen. Dadurch können vorteilhafterweise höhere Umsätze erreicht und Produktaufarbeitung sowie Katalysatorrecycling erheblich vereinfacht werden.

Organometallische Katalysatoren sind Katalysatoren, in denen organische Reste direkt an ein Metallatom gebunden sind. Bekannte Vertreter der organometallischen Katalysatoren sind die Grubbs-Katalysatoren (Ruthenium-Carben-Komplexe, z.B. Grubbs I (Benzylidenbis(tricyclohexylphosphin)dichlororuthenium)), die vielfach in der Olefinmetathese Anwendung finden. Im Gegensatz zu anderen Olefinmetathese-Katalysatoren ist der Grubbs-Katalysator unempfindlich gegenüber funktionellen Gruppen im Substrat und vielseitig einsetzbar in einem weiten Spektrum von Lösungsmitteln. Ausgehend von der ersten Generation an Grubbs-Katalysatoren führten mehrere Optimierungen zu einer Vielzahl von verfügbaren Katalysatoren wie z.B. von modifizierten Katalysatoren erster Generation, NHC (*N*-heterozyklischen Carben)-Liganden anstelle von Triphenylphosphin (Grubbs II), chelatierten NHC-Liganden, zweizähnigen NHCs und verbrückenden Aryloxy-Liganden bis zu Thiolat-modifizierten Grubbs-Hoveyda-Katalysatoren zweiter Generation (Grubbs-Hoveyda-Katalysatoren unterscheiden sich von den herkömmlichen Grubbs-Katalysatoren dadurch, dass an einer Position ein Tricyclohexylphosphin-Ligand entfernt wurde und die freie Koordinationsstelle von einem aromatischen Ether besetzt wird, der aus dem carbengebundenen Rest stammt) und chiralen Grubbs-Katalysatoren.

Der Einbau bestimmter Grubbs-Hoveyda-Katalysatoren in das vorliegende, erfindungsgemäße Biohybridkatalysator-System ist besonders vorteilhaft, da NHC-Ruthenium-Katalysatoren eine erhöhte Stabilität gegenüber Feuchtigkeit bzw. Wasser, Luft bzw. Sauerstoff und eine höhere Toleranz gegenüber funktionellen Gruppen zeigen. Zudem ist die Metathese eine bioorthogonale Reaktion. Des Weiteren erlaubt die Stabilität dieser Metallkomplexe vorteilhafterweise Modifikationen am NHC-Liganden des Katalysators, wie z.B. die Anbringung von verschiedenen Linkern zur (vorzugsweise kovalenten) Anbindung des Katalysators an biologische (Protein-)Gerüste.

Die Rh-Carben-Katalysatoren und Rh-Cyclopentadienyl-Katalysatoren weisen eine hohe Stabilität gegenüber Luft bzw. Sauerstoff und Luftfeuchtigkeit bzw. Wasser auf. Zudem weisen sie ein breites Substratspektrum auf und können funktionelle Gruppen tolerieren. Komplexe basierend auf Cyclopentadienyl-Liganden sind stabil gegenüber Wasser. Sie sind klein und können daher sehr einfach in ein Protein eingebunden werden. Zudem bietet der Ligand Möglichkeiten zur Modifikation. Terpyridin-Komplexe sind von der Geometrie ähnlich zur natürlichen Hämgruppe. Platin ist biologisch verträglich und kann in Terpyridin-Komplexe eingebunden werden und ermöglicht somit die Verankerung an Proteinen zur Katalysator-Entwicklung. Pd-Carben-Katalysatoren sind in Wasser stabil und zeigen erhöhte Aktivität. Die Beeinflussung durch eine Proteinumgebung zeigt zudem Einfluss auf die Selektivität des aktiven Zentrums. Vollständige Entfernung des Metalls nach der Reaktion kann durch Anbindung an das Protein gelingen. Allgemein tolerieren die genannten Katalysatoren andere Metalle/Enzyme, sodass Kaskaden möglich werden.

Die Fähigkeit, verschiedenste Arten von katalytisch aktiven Metallkomplexen an das Proteingerüst bzw. das erfindungsgemäße Biohybridkatalysator-System anbinden zu können, ist besonders vorteilhaft, da das erfindungsgemäße Biohybridkatalysator-System dadurch zur Katalyse verschiedenster Reaktionen - wie z.B. von Metathese-Reaktionen, Acetylen-Trimerisierungen und -Polymerisierungen, Diels-Alder-Reaktionen, Hydrogenierungen und Lactidpolymerisationen - eingesetzt werden kann.

Eine weitere bevorzugte Ausführungsform betrifft ein Biohybridkatalysator-System wie vorangehend definiert, wobei das/die Metalloenzym(e) über Zelloberflächendisplay an der Zelloberfläche der Zelle angebunden ist/sind.

Zelloberflächendisplay-Systeme werden zur Expression von Proteinen und Peptiden auf der Zelloberfläche von Prokaryoten (z.B. Bakterien) und Eukaryoten (z.B. Hefen und Säugetierzellen) verwendet. Die erfindungsgemäße Anbindung des/der künstlichen Metalloenzyms/enzyme über Zelloberflächendisplay an der Zelloberfläche der Zelle ist besonders vorteilhaft, da die gezeigten künstlichen Metalloenzyme auf der Zelloberfläche für das Substrat der Katalysereaktion frei zugänglich sind. Mit der Zellhülle als Matrix sind Proteine oder Peptide wie die erfindungsgemäßen künstlichen Metalloenzyme des Weiteren in der Regel stabiler; eine Aufreinigung ist dabei im Regelfall nicht notwendig.

Ein weiterer Aspekt der vorliegenden Erfindung betrifft ein Verfahren zur Herstellung eines Biohybridkatalysator-Systems wie vorangehend definiert, umfassend folgende Schritte:
(ii.1) Bereitstellen eines Proteingerüsts, das Bestandteil einer intakten, nicht-menschlichen Zelle, vorzugsweise Bestandteil der Zellmembran, beispielsweise der äußeren Zellmembran, und/oder der Zelloberfläche einer intakten, nicht-menschlichen Zelle ist, und
(ii.2) Anbinden eines katalytisch aktiven Metallkomplexes, enthaltend ein Übergangsmetall, vorzugsweise ein Metall ausgewählt aus der Gruppe bestehend aus Ruthenium, Rhodium, Cobalt, Kupfer, Iridium, Mangan, Palladium und Platin, an das Proteingerüst, vorzugsweise mithilfe dativer oder supramolekularer Wechselwirkungen oder mithilfe kovalenter Bindungen,
wobei der Metallkomplex in Schritt (ii.2) ausgewählt ist aus der Gruppe bestehend aus organometallischen Katalysatoren, vorzugsweise Grubbs-Hoveyda-Katalysatoren, Rh-Cyclopentadien-Katalysatoren, Rh-Carben-Katalysatoren, Cu-Carben-Katalysatoren, Cu-Terpyridin-Katalysatoren, Cu-Phenanthrolin-Katalysatoren, Cu-Pyren- und Co-Terpyridin-Katalysatoren, Pt-Terpyridin-Katalysatoren, Pt-Carben-Katalysatoren, Pd-Terpyridin-Katalysatoren, Pd-Carben-Katalysatoren, Ir-Carben-Katalysatoren und Mn-Cyclopentadienyl-Katalysatoren.

Um die Präsenz des katalytisch aktiven Metallkomplexes in der Bindungstasche des Proteingerüsts sicherzustellen, werden bevorzugt die folgenden drei Konzepte verwendet:
(a) Dative Koordination des Metall-Atoms des Metallkomplexes durch Aminosäure-Reste des Proteingerüsts,
(b) Supramolekulare Anbindung des katalytisch aktiven Metallkomplexes oder dessen Substituenten durch die Proteinumgebung des Proteingerüsts, und/oder
(c) Kovalente Bindung des vorzugsweise funktionalisierten, katalytisch aktiven Metallkomplexes an das Proteingerüst.

Das Anbinden des katalytisch aktiven Metallkomplexes an das Proteingerüst mithilfe kovalenter Bindungen ist besonders vorteilhaft, da z.B. durch die kovalente Anbindung des katalytisch aktiven Metallkomplexes an die nukleophile Thiol-Funktion eines Cysteins des Proteingerüsts eine hoch ortsspezifische und stabile Anbindung des katalytisch aktiven Metallkomplexes an das Gerüstprotein erfolgt. Thiol-reaktive Gruppen binden vorzugsweise durch Alkylierung zu einer Thioetherbindung oder Disulfidaustausch an Cystein (z.B. Halogenacetate, Maleimide, Aziridine, Acrylchloride, Vinylsulfone and Pyridyldisulfide). Der Einbau nicht-kanonischer Aminosäuren (z.b. Homopropargylglycine, Homoallylglycine) durch neue orthogonale Aminoacyl-tRNA Synthetasen (aaRS)/tRNA-Paare ermöglicht zusätzliche Anbindungen von Katalysatorkomplexen mittels Click-Chemie bis hin zu Mehrpunktverankerungen, die zu einer deutlich besser definierten Katalysatorlokalisierung mit erhöhter Enantioselektivität führen.

Eine weitere bevorzugte Ausführungsform betrifft ein Verfahren wie vorangehend definiert, wobei die Zelle in Schritt (ii.1) ausgewählt ist aus der Gruppe bestehend aus Bakterienzellen, vorzugsweise gram-negativen Bakterienzellen, wie *E. coli* und *Pseudomonas.*

Der Einsatz gram-negativer Bakterienzellen als intakte, nicht-menschliche Zelle im vorangehend definierten, erfindungsgemäßen Verfahren ist besonders vorteilhaft, da diese sehr gut erforschte und etablierte Zellsysteme sind, die durch ihren strukturellen Aufbau zudem besonders gut geeignet sind für die funktionale Einlagerung (d.h. Verankerung oder Anbindung) der Gerüstproteine in die äußere Zellmembran.

Wie vorangehend erwähnt, eignen sich neben gram-negativen Bakterienzellen für das erfindungsgemäße Biohybridkatalysator-System prinzipiell ebenso gram-positive Bakterien, wie *Bacillus*-Stämme, da gram-positive Bakterien mit ihrer Cytoplasmamembran ebenfalls über eine Zellmembran verfügen. Im Rahmen der vorliegenden Erfindung können beispielsweise Corynebakterien als Zellen verwendet werden.

Eine weitere bevorzugte Ausführungsform betrifft ein Verfahren wie vorangehend definiert, wobei das Proteingerüst in Schritt (ii.1) ausgewählt ist aus der Gruppe bestehend aus Transmembranproteinen und löslichen Proteinen, vorzugsweise solchen mit beta-Fassstruktur, vorzugsweise solchen mit 6 bis 24 beta-Faltblättern, bevorzugt FhuA oder Nitrobindin, membranständigen oder verankerten Proteinen, vorzugsweise LCI.

Der Einsatz von Transmembranproteinen und löslichen Proteinen, vorzugsweise solchen mit beta-Fassstruktur, insbesondere der Einsatz des Transmembranproteins FhuA und des löslichen Proteins Nitrobindin als Proteingerüst im erfindungsgemäßen Verfahren ist besonders vorteilhaft, da er durch die großen Kavitäten der Gerüstproteine eine Positionierung der katalytisch aktiven Metallkomplexe tief in der Fassstruktur der Transmembranproteine und nicht nur an der Proteinoberfläche ermöglicht. Die tiefe Positionierung führt zu einer umfassenden Kontrolle der Umgebung des katalytisch wirksamen Metallkomplexes durch spezifisch austauschbare Aminosäuren des Proteingerüsts und damit zur Steuerung der Selektivität des Metallkomplexes.

Der Einsatz organometallischer Katalysatoren, wie z.B. von bestimmten Grubbs-Hoveyda-Katalysatoren im erfindungsgemäßen Verfahren wie vorangehend definiert, ist besonders vorteilhaft, da NHC-Ruthenium-Katalysatoren eine erhöhte Stabilität gegenüber Feuchtigkeit bzw. Wasser, Luft bzw. Sauerstoff und eine höhere Toleranz gegenüber funktionellen Gruppen zeigen. Des Weiteren erlaubt die Stabilität dieser Metallkomplexe vorteilhafterweise Modifikationen am NHC-Liganden des Katalysators, wie z.B. die Anbringung von verschiedenen Linkern zur (vorzugsweise kovalenten) Anbindung des Katalysators an biologische (Protein-)Gerüste.

Ein weiterer Aspekt der vorliegenden Erfindung betrifft ein Biohybridkatalysator-System wie vorangehend definiert, hergestellt oder herstellbar durch ein Verfahren wie vorangehend definiert.

Ein weiterer Aspekt der vorliegenden Erfindung betrifft zudem ein Verfahren zur Katalyse einer chemischen Reaktion, umfassend folgende Schritte:
(a) Bereitstellen eines Biohybridkatalysator-Systems wie vorangehend definiert, und
(b) Katalyse einer chemischen Reaktion unter Verwendung des Biohybridkatalysator-Systems, wobei die Reaktion durch die katalytische Aktivität des an das Proteingerüst angebundenen, katalytisch aktiven Metallkomplexes katalysiert wird.

Die chemische Reaktion kann dabei gemäß der vorliegenden Erfindung in wässrigem Medium, in organischen Lösungsmittel und/oder reinem Substrat erfolgen.

Die Katalyse einer chemischen Reaktion unter Verwendung des erfindungsgemäßen Biohybridkatalysator-Systems ist besonders vorteilhaft, da durch das erfindungsgemäße Ganzzell-Biohybridkatalysator-System chemische Umsetzungen in der industriellen Stoffproduktion realisiert werden können, die z.B. mit isolierten Biohybridkatalysatoren insbesondere aus Kostengründen nicht attraktiv sind.

Die erfindungsgemäßen Biohybridkatalysatoren stellen vorteilhafterweise eine spezifische Umgebung für die angebundenen, katalytisch aktiven Metallkomplexe bereit und ermöglichen so die Nutzung des natürlich nicht ausgeschöpften Synthesepotentials seltener Metalle (Ruthenium, Rhodium) in Enzymen. Durch Verwendung von beta-Fassstruktur-Proteinen wie FhuA oder Nitrobindin als Umgebung für die katalytisch aktiven Metallkomplexe können erstmals Hochdurchsatzdurchmusterungsmethoden genutzt werden, um eine generierte Vielfalt an Biohybridvarianten durchzumustern. Mittels kovalenter Anbindung der Metallkomplexe an die Proteinumgebung wird dabei die definierte Bindung und Orientierung der Metallkomplexe innerhalb der Proteinumgebung realisiert, auch bei umfassenden Änderungen der Umgebung mittels Proteinengineering.

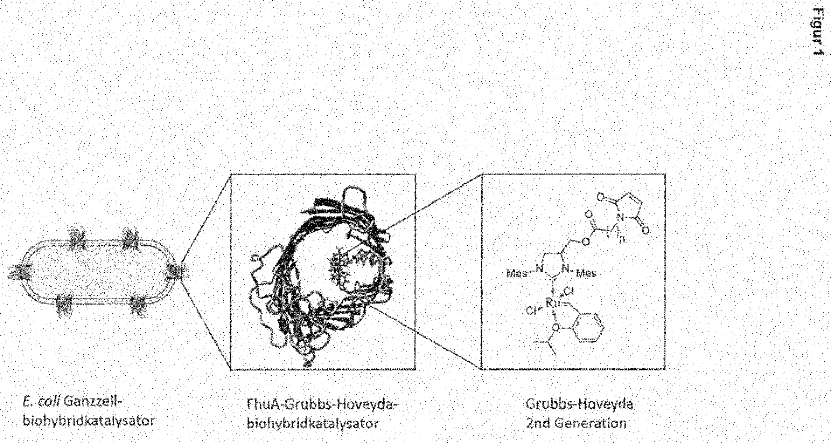

Wie in Figur 1 gezeigt, nutzt die Anwendung von beta-Fassstruktur-Membranproteinen oder membranverankerten Fass-Proteinen die Zellmembran als natürliche Barriere, um inhibierende Bestandteile aus dem Cytosol der Zellen innerhalb der Zellen zu halten. Somit wird die ungewollte Interaktion dieser schädlichen Nukleophile mit den katalytisch aktiven Metallkomplexen innerhalb der Biohybridkatalysatoren verhindert.

Die Bakterienzellen des erfindungsgemäßen Biohybridkatalysator-Systems werden bei schonender Lyophilisierung und Rehydratisierung intakt gehalten und eventuell vorhandene inhibierende Substanzen können einfach ausgewaschen werden. Durch die Immobilisierung an oder innerhalb der Zellmembran lässt sich eine Trennung von freiem (nicht angebundenem / unfixiertem) Metallkomplex vom Biohybridkatalysator bzw. die Abtrennung des Biohybridkatalysators vom Reaktionsprodukt leicht erreichen (z.B. durch Zentrifugieren) und ermöglicht zusätzlich eine maximale Zugänglichkeit der Substrate zu den aktiven Komponenten.

Insbesondere die Expression als Membranprotein in der äußeren Zellmembran ermöglicht das Durchführen von Katalyse-Reaktionen unter Bedingungen, die mit dem isolierten, im Reaktionsmedium gelösten Protein aufgrund von Stabilitätsproblemen nicht möglich wären, da das Membranprotein durch die natürliche Membranumgebung stabilisiert wird.

Eine bevorzugte Ausführungsform betrifft ein Verfahren wie vorangehend definiert, wobei die chemische Reaktion ausgewählt ist aus der Gruppe bestehend aus Metathese, Acetylen-Trimerisierung und -Polymerisierung, Diels-Alder-Reaktionen, Hydrogenierungen und Lactidpolymerisationen.

Durch die Fähigkeit, verschiedenste Arten von katalytisch aktiven Metallkomplexen an das Proteingerüst bzw. das erfindungsgemäße Biohybridkatalysator-System anbinden zu können, besteht vorteilhafterweise die Möglichkeit, das erfindungsgemäße Biohybridkatalysator-System zur Katalyse für ein breites Spektrum verschiedener chemischer Reaktionen einsetzen zu können.

Eine weitere bevorzugte Ausführungsform betrifft ein Verfahren wie hierin beschrieben, zudem umfassend, vorzugsweise zwischen Schritt (a) und Schritt (b), folgenden Schritt:
- Immobilisierung der intakten, nicht-menschlichen Zelle des Biohybridkatalysator-Systems wie hierin beschrieben an der Oberfläche eines Objekts, z. B. von Beads.

Die Immobilisierung der Ganzzellbiohybridkatalysatoren verringert die Kosten von (bio)katalytischen Prozessen erheblich durch die mögliche kontinuierliche Arbeitsweise und die Aufrechterhaltung hoher Zelldichten. Durch Immobilisierung der Ganzzellbiohybridkatalysatoren kann weiterhin die Wirkung von Scherkräften auf die Zellen verringert werden, was die Beschädigung der Zellhülle und Freisetzung Katalysator-deaktivierender Zellbestandteile unterbindet.

Die Immobilisierung kann vorteilhafterweise sehr einfach durch vorzugsweise Zelloberflächen-Display-Expression von oberflächenbindenden Peptiden (z.B. LCI) realisiert werden.

Eine weitere bevorzugte Ausführungsform betrifft ein Verfahren wie vorangehend definiert, zudem umfassend folgenden Schritt:
- Abtrennen des Biohybridkatalysator-Systems von dem/den Reaktionsprodukt(en) der chemischen Reaktion.

Die Abtrennung des erfindungsgemäßen Biohybridkatalysator-Systems kann vorteilhafterweise sehr einfach und effizient durch vorzugsweise Zentrifugieren oder manuelle Abtrennung des erfindungsgemäßen, immobilisierten Biohybridkatalysator-Systems erfolgen. Vorteilhafterweise muss das erfindungsgemäße Biohybridkatalysator-System zur Gewinnung der Reaktionsprodukte daher nicht zerstört werden und kann somit gegebenenfalls für weitere Katalysereaktionen wiederverwendet werden.

Ein weiterer Aspekt der vorliegenden Erfindung betrifft die Verwendung eines Biohybridkatalysator-Systems wie vorangehend definiert als Katalysator zur Katalyse einer chemischen Reaktion, vorzugsweise zur Katalyse einer Reaktion wie vorangehend definiert.

Ein weiterer Aspekt der vorliegenden Erfindung betrifft die Verwendung eines Biohybridkatalysator-Systems wie vorangehend definiert als Katalysator zur Katalyse einer chemischen Reaktion, vorzugsweise zur Katalyse einer Reaktion wie vorangehend definiert, in wässrigen Systemen, als auch in organischem Lösungsmittel und/oder reinem Substrat als Lösungsmittel.

Das im Rahmen einer hierin beschriebenen Ausführungsform der vorliegenden Erfindung Gesagte gilt selbstverständlich jeweils auch für andere hierin beschriebene Ausführungsformen. Dementsprechend sind die hierin beschriebenen Ausführungsformen beliebig - soweit für den Fachmann sinnvoll - miteinander kombinierbar.

Kurze Beschreibung der Figur:
**Figur 1** zeigt eine schematische Darstellung eines beispielhaften, erfindungsgemäßen Ganzzell-Biohybridkatalysator-Systems. Der katalytisch aktive Metallkomplex (hier beispielhaft ein Grubbs-Hoveyda-II-Katalysator) ist an ein beta-Fassstruktur-Membranprotein (hier beispielhaft FhuA) gebunden, welches sich in der Zellmembran der Bakterienzelle (hier beispielhaft *E. coli*) befindet.

### Sequenzprotokoll - freier Text

Die folgenden Angaben geben die deutsche Übersetzung der im Sequenzprotokoll als freier Text (Numerische Kennzahl <223>) enthaltenen Angaben, jeweils aufgeführt für die entsprechende Sequenzkennzahl, wieder. Alle angeführten Sequenzen enthalten unter der Kennzahl <213> den Hinweis auf eine künstliche Sequenz ("artificial sequence").
SEQ ID NO: 1 bis 18
<223>

### Künstliches Molekül ("artificial molecule")

### Definitionen

- ROMP: Ringöffnungsmetathese Polymerisation
- RCM: Ringschlussmetathese
- CM: Crossmetathese
- HPLC: Hochleistungsflüssigkeitschromatographie
- NaPi: Natriumphosphat-Puffer
- LCI: Antimikrobielles Peptid LCI aus *B*. *subtilis* A014
- TEV: Tobacco etch virus (Tabakätzvirus)
- THF: Tetrahydrofuran
- DCM: Dichlormethan
- EtOAc: Ethylacetat
- ATCC: American Type Culture Collection
- PBS: Phosphatgepufferte Salzlösung

Im Folgenden wird die vorliegende Erfindung anhand ausgewählter Beispiele näher erläutert. Sofern nicht anders angegeben, beziehen sich dabei alle Prozentangaben auf das Gewicht (Gew.-%).

### Beispiel 1: E. coli Biohybridkatalysator-Systeme mit FhuA ΔCVF^{TEV} als Proteingerüst

### 1. Synthese katalytisch aktiver Metallkomplexe:

### 1.a) Synthese von Rh-1,5-cyclooctadienyl-cyclopentadienyl-ethyl-maleimid [RhCOD-Katalysator]:

Cyclooctadien Rhodium Chlorid Dimer [Rh(cod)Cl]₂ (79 mg, 0,16 mmol) wird in THF (3 mL) vorgelegt. Zu dieser Lösung wird eine Lösung aus 2-(cyclopentadienyl)-ethan-1-amin Lithium C₇H₁₀NLi (40 mg, 0,37 mmol) in 3 mL THF zugetropft. Die Lösung wird für 30 Minuten bei 25 °C gerührt. Anschließend wird N-Methyoxcarbonylmaleimid (C₆H₅NO₄) (55 mg, 0,37 mmol) in 10 mL THF langsam zugetropft, und die Reaktionsmischung für 24 Stunden bei 25 °C gerührt. Nach beendeter Reaktion wird Wasser (30 mL) zugegeben, und die Lösung mit DCM (3 x 30 mL) ausgeschüttelt. Das organische Lösungsmittel wird im Vakuum entfernt und der Rückstand säulenchromatographisch aufgereinigt (Al₂O₃, THF). Der Rh-Katalysator (C₂₁H₂₆NO₂Rh) wird als oranges Pulver (ca. 50 mg) erhalten. (Vgl. hierzu Onoda, A., Fukumoto, K., Arlt, M., Bocola, M., Schwaneberg U., Hayashi, T., A rhodium complex-linked β-barrel protein as a hybrid biocatalyst for phenylacetylene polymerization", Chem. Comm., 2012, 48, 9756-9758.)

### 1.b) Synthese von ([1,3-Bis(1-mesityl)-4-(methyl-N-maleimidopropansäure-ester)-4,5-dihydroimidazol-2-yliden][2-isopropoxybenzyliden]ruthenium(IV)-dichlorid) [Grubbs-Hoveyda-II mit Maleimid-Gruppe]:

1,3-Dimesityl-4,5-dihydro-4-(tetrahydropyranyl)-1*H*-imidazol-3-ium-chlorid (503 mg, 1,10 mmol) wurde in THF (5 mL) aufgenommen und mit Kaliumhexamethyldisilazid (220 mg, 1,10 mmol) versetzt. Diese Lösung wurde zu einer Lösung des Grubbs-Katalysator der ersten Generation (823 mg, 1,00 mmol, 1,00 Äquiv.) in THF (10 mL) zugefügt. Das Reaktionsgemisch wurde für 16 h bei 60 °C gerührt. Das Lösungsmittel wurde unter Vakuum entfernt, der rote Rückstand in Benzol (3 mL) aufgenommen und säulenchromatographisch an Kieselgel gereinigt (Lösungsmittel: n-Pentan/Et₂O - 2:1). Der Grubbs-Katalysator der zweiten Generation (Tricyclohexylphosphin[1,3-bis(1-mesityl)-4-tetrahydropyranyl-4,5-dihydroimidazol-2-yliden][benzyliden]ruthenium(IV)-dichlorid) wurde als roter Feststoff erhalten.

Tricyclohexylphosphin[1,3-bis(1-mesityl)-4-tetrahydropyranyl-4,5-dihydroimidazol-2-yliden][benzyliden]ruthenium(IV)-dichlorid (860 mg, 0,891 mmol) und Kupfer(I)-Chlorid (133 mg, 1,34 mmol) wurden in DCM (8 mL) aufgenommen. Zu der roten Lösung wurde eine Lösung aus 2-Isopropoxystyrol (182 mg, 1,13 mmol) in DCM (2 mL) zugetropft. Das Reaktionsgemisch wurde für 3 h bei 40 °C gerührt, bis die Lösung grün gefärbt war. Das Lösungsmittel wurde unter Vakuum entfernt und der braun-grüne Rückstand in Pentan/DCM (2:1) aufgenommen und über Celite filtriert. Das Lösungsmittel wurde eingeengt und die braun-grüne Lösung säulenchromatographisch an Kieselgel (Lösungsmittel: erst DCM, dann EtOAc) gereinigt. Der geschützte Grubbs-Hoveyda Katalysator der zweiten Generation ([1,3-bis(1-mesityl)-4-tetrahydropyranyl-4,5-dihydroimidazol-2-yliden][2-isopropoxybenzyliden]ruthenium(IV)-dichlorid) wurde als grüner Feststoff (550 mg, 0,74 mmol) erhalten.

Eine Lösung aus [1,3-bis(1-mesityl)-4-tetrahydropyranyl-4,5-dihydroimidazol-2-yliden][2-isopropoxybenzyliden]ruthenium(IV)-dichlorid (550 mg, 0,74 mmol) in Ethanol (20 mL) wurde mit wässriger HCl-Lösung (1,0 M, 0,30 mL) versetzt und für 20 min mit Argon entgast. Die Lösung wurde unter Argonatmosphäre für 72 h gerührt. Das Lösungsmittel wurde unter Vakuum entfernt. Der grüne Rückstand wurde in DCM (20 mL) aufgenommen und mit Wasser (2 × 20 mL) gewaschen. Die org. Phase wurde über Na₂SO₄ getrocknet, das Lösungsmittel unter Vakuum entfernt und der grün-braune Rückstand säulenchromatographisch an Kieselgel (Lösungsmittel: DCM) gereinigt. Der Grubbs-Hoveyda Katalysator der zweiten Generation ([1,3-Bis(1-mesityl)-4-methylhydroxy-4,5-dihydroimidazol-2-yliden][2-isopropoxybenzyliden]ruthenium(IV)-dichlorid) konnte als grüner Feststoff (350 mg, 0,533 mmol) erhalten werden.

([1,3-Bis(1-mesityl)-4-methylhydroxy-4,5-dihydroimidazol-2-yliden][2-isopropoxybenzyliden]ruthenium(IV)-dichlorid) (30 mg, 0,05 mmol) wurde in THF (0,5 mL) vorgelegt. Eine äquimolare Menge des *N*-Maleimidopropansäurechlorids (13 mg, 0,05 mmol) wurde in THF (0,5 mL) zugetropft. Zu der Lösung wurde NaHCO₃ (30 mg, 0,35 mmol) zugegeben. Das Reaktionsgemisch wurde für 24 h bei 25 °C gerührt. Anschließend wurde die Lösung filtriert und mit Et₂O (15 mL) verdünnt. Die Lösung wurde mit Wasser (3 x 15 mL) gewaschen und die org. Phase über Na₂SO₄ getrocknet und das Lösungsmittel unter Vakuum entfernt. Der Grubbs-Hoveyda-Katalysator der zweiten Generation mit Maleimid-Gruppe ([1,3-Bis(1-mesityl)-4-(methyl-N-maleimidopropansäure-ester)-4,5-dihydroimidazol-2-yliden][2-isopropoxybenzyliden] ruthenium(IV)-dichlorid) wurde als grüner Feststoff (35 mg, 0,047 mmol) erhalten. (Vgl. hierzu Philippart, F., Arlt, M., Gotzen, S., Tenne, S.-J., Bocola, M., Chen, H.-H., Zhu, L., Schwaneberg, U. und Okuda, J., A Biohybrid Ring-Opening Metathesis Polymerization Catalyst Based on an Engineered Variant of the β-Barrel Protein FhuA, Chem. Eur. J., 2013, 19, 13865-13871.)

### 1.c) Synthese von [1-{(1-Mesityl)-3-(2,5-dioxo-2,5-dihydro-1H-pyrrol-1-yl)propyl)}-4,5-dihydroimidazol-3-yliden]kupfer(I)-iodid[Cu-Carben-Komplex]

1-{(1-Mesityl)-3-(2,5-dioxo-2,5-dihydro-1H-pyrrol-1-yl)propyl)}-4,5-dihydro-1H-imidazol-3-ium-bromid (100 mg, 0,247 mmol) wurde in THF (2 mL) suspendiert und Kalium-*tert*-butanolat (28.0 mg, 0,249 mmol) in THF (3 mL) zugetropft. Es wurde für 10 min gerührt. Das Reaktionsgemisch wurde mit Cul (48.0 mg, 0,252 mmol, 1,00 Äquiv.) versetzt und bei 23 °C für 12 h gerührt. Es wurde über Kieselgur filtriert und das Lösungsmittel unter Vakuum entfernt. [1-{(1-Mesityl)-3-(2,5-dioxo-2,5-dihydro-1H-pyrrol-1-yl)propyl)}-4,5-dihydroimidazol-3-yliden]kupfer(I)-iodid wurde als brauner Feststoff erhalten (82 mg, 0,15 mmol). (Vgl. hierzu Osseili, H., Sauer, D., Beckerle, K., Arlt, M., Himiyama, T., Polen, T., Onoda, A., Schwaneberg, U., Hayashi, T., Okuda, J., Artificial Diels-Alderase based on the transmembrane protein FhuA, Beilstein J. Org. Chem. 2016, 12, 1314-1321.)

### 2. Bereitstellung des Expressionsstamms E. coli BL21 (DE3) Omp8 (F⁻ hsdS_{B} (r_{B}⁻m_{B}⁻) gal ompT dcm (DE3) ΔlamB ompF::Tn5 ΔompA ΔompC), ausgehend von E. coli BL21 (DE3) (Novagen):

Durch die zusätzliche Entfernung von Membranproteinen aus dem *E. coli* Genom kann eine höhere Ausbeute an membranexprimiertem bzw. zelloberflächendargestelltem FhuA erreicht werden. Dazu wurde ein Expressionsstamm entwickelt, bei dem die Hauptmembranproteine LamB, OmpA, OmpC, OmpF durch P1 Transduktion mit bekannten Veränderungen der Stämme BZB1107 (ompR, vlamB, ompF: :Tn5), BRE2413 (vompA), and RAM191 (vompC) mutiert wurden. (Vgl. hierzu Prilipov, A.; Phale, P. S.; Van Gelder, P.; Rosenbusch, J. P.; Koebnik, R., Coupling site-directed mutagenesis with high-level expression: large scale production of mutant porins from E. coli. FEMS Microbiology Letters 1998, 163(1), 65-72).

### 3. Design und Herstellung des Proteingerüsts (hier: FhuA Variante FhuA ΔCVF^{TEV} (SEQ ID No: 1 / SEQ ID No: 2)):

Ausgehend vom FhuA WT (pdb: 1BY3, Locher, K. P.; Rees, B.; Koebnik, R.; Mitschier, A.; Moulinier, L.; Rosenbusch, J. P.; Moras, D., , Cell 95 (1998) 771-778) wurden die Aminosäuren 1-60 deletiert und an Position 545 Lysin gegen Cystein ausgetauscht (Nallani, M.; Onaca, O.; Gera, N.; Hildenbrand, K.; Hoheisel, W.; Schwaneberg, U., Biotechnology Journal 1 (2006) 828-834). Durch Austausch von Asparagin zu Valin an Position 548 und Glutaminsäure zu Phenylalanin an Position 501 konnten Cystein-Zugänglichkeit und Katalysatorkompatibilität verbessert werden. Die TEV-Schnittstellen ENLYFQ|G wurden eingefügt in den Loops 7 (zwischen Asp509 und Lys508) und 8 (zwischen Asn548 und Glycine549).

Das synthetische Gen mit den oben beschriebenen Modifikationen wurde mittels Restriktionsklonierung (EcoRI und Xhol) in den Expressionsvektor pBR-IBA1 eingeführt. FhuA wurde in den Expressionswirt *E. coli* BL21 (DE3) omp8 mittels Hitzeschock transformiert. Die Expression wurde durchgeführt in LB-Medium, enthaltend 1 mM Ampicillin, als Vorkultur und TY als Hauptkultur bei 30 °C für 12 h mit Isopropyl-β-D-1-thiogalactopyranosid (IPTG, 1 mM), um die Expression zu induzieren. Nach Übernachtexpression wurde die Kultur durch Zentrifugation geerntet. (Vgl. hierzu Philippart, F., Arlt, M., Gotzen, S., Tenne, S.-J., Bocola, M., Chen, H.-H., Zhu, L., Schwaneberg, U. und Okuda, J., A biohybrid Ring-Opening Metathesis Polymerization Catalyst Based on an Engineered Variant of the β-Barrel Protein FhuA, Chem. Eur. J., 2013, 19, 13865-13871).

### 4. Anbinden der katalytisch aktiven Metallkomplexe an das Proteingerüst:

Das geerntete Pellet wurde mit ddH₂O/Glyzerin gewaschen und in flüssigem Stickstoff eingefroren. Die Zellen wurden anschließend lyophilisiert und in PBS-Puffer (pH 7,5) rehydriert. Die jeweiligen Metallkomplexe (s.o., Bsp. 1.a, 1.b, 1.c) wurden *via* Maleimid und Cystein gekoppelt mit 10 Äquivalenten Katalysator in PBS-Puffer (pH 7,5) und 20% (v/v) THF als Co-Lösungsmittel. Die Suspension wurde 24 h bei Raumtemperatur gerührt, gereinigt, zentrifugiert (4000 rpm, 10 min) und mit PBS/THF (1:1) mindestens 5 Mal gewaschen. Die Waschlösungen wurden mithilfe von HPLC-Chromatographie untersucht, um die Entfernung von nicht gebundenem Katalysator zu bestätigen. (Als Negativkontrolle wurde als Proteingerüst die Variante FhuA Δ1-160 (SEQ ID No: 3; SEQ ID No: 4) ohne frei verfügbares Cystein verwendet.)

### 5. Katalytische Reaktionen mit erfindungsgemäßen Biohybridkatalysator-Systemen umfassend Rh, Ru, Cu-Metallkomplexe

### 5.a) Phenylacetylen-Polymerisierung und -Trimerisierung:

Für die Phenylacetylen-Polymerisierung wurden 5 ml einer Zellsuspension aus Biohybridkatalysator-Systemen mit RhCOD-Katalysator (s.o., Bsp. 1.a) in 10 mM NaPi (pH 8), entsprechend einer molaren Katalysatorladung von 0,1 Mol%, bereitgestellt und mit 15 µl Monomer (Phenylacetylen) versetzt. Nach 48 h Rühren bei 25 °C wurden die Produkte isoliert und mittels NMR-Spektroskopie identifiziert.

### 5.b) Metathese (ROMP, RCM, CM):

Für die ROMP wurden 5 ml einer Zellsuspension aus Biohybridkatalysator-Systemen mit Grubbs-Hoveyda-II-Katalysator mit Maleimid-Gruppe (s.o., Bsp. 1.b) in 10 mM NaPi (pH 5,8) mit 20% (v/v) THF, entsprechend einer molaren Katalysatorladung von 0,1 Mol%, bereitgestellt und mit 15 µl Monomer (7-Oxanorbonen) versetzt. Nach 68 h Rühren bei 25 °C wurden die Produkte isoliert und mittels NMR-Spektroskopie identifiziert.

### 5.c) Diels-Alder-Reaktionen:

Für die Diels-Alder-Reaktion von Azochalcon und Cyclopentadien wurden 5 ml einer Zellsuspension aus Biohybridkatalysator-Systemen mit Cu-Carben-Komplex-Katalysator (s.o., Bsp. 1.c) in 100 mM NaPi (pH 7,5) mit 20% (v/v) THF, ensprechend einer molaren Katalysatorladung von 1 Mol%, bereitgestellt und mit Azochalcon und Cyclopentadien versetzt. Nach 72 h Rühren bei 23 °C wurden die Produkte isoliert und mittels NMR und HPLC identifiziert.

### Beispiel 2: Pseudomonas putida Biohybridkatalysator-Systeme mit FhuA ΔCVF^{TEV} als Proteingerüst

### 1. Synthese katalytisch aktiver Metallkomplexe:

### Synthese von ([1,3-Bis(1-mesityl)-4-(methyl-N-maleimidopropansäure-ester)-4,5-dihydroimidazol-2-yliden][2-isopropoxybenzyliden]-ruthenium(IV)-dichlorid) [Grubbs-Hoveyda-II mit Maleimid-Gruppe]:

1,3-Dimesityl-4,5-dihydro-4-(tetrahydropyranyl)-1*H*-imidazol-3-ium-chlorid (503 mg, 1,10 mmol) wurde in THF (5 mL) aufgenommen und mit Kaliumhexamethyldisilazid (220 mg, 1,10 mmol) versetzt. Diese Lösung wurde zu einer Lösung des Grubbs-Katalysator der ersten Generation (823 mg, 1,00 mmol, 1,00 Äquiv.) in THF (10 mL) zugefügt. Das Reaktionsgemisch wurde für 16 h bei 60 °C gerührt. Das Lösungsmittel wurde unter Vakuum entfernt, der rote Rückstand in Benzol (3 mL) aufgenommen und säulenchromatographisch an Kieselgel gereinigt (Lösungsmittel: n-Pentan/Et₂O - 2:1). Der Grubbs-Katalysator der zweiten Generation (Tricyclohexylphosphin[1,3-bis(1-mesityl)-4-tetrahydropyranyl-4,5-dihydroimidazol-2-yliden][benzyliden]ruthenium(IV)-dichlorid) wurde als roter Feststoff erhalten.

Tricyclohexylphosphin[1,3-bis(1-mesityl)-4-tetrahydropyrany1-4,5-dihydroimidazol-2-yliden][benzyliden]ruthenium(IV)-dichlorid (860 mg, 0,891 mmol) und Kupfer(I)-Chlorid (133 mg, 1,34 mmol) wurden in DCM (8 mL) aufgenommen. Zu der roten Lösung wurde eine Lösung aus 2-Isopropoxystyrol (182 mg, 1,13 mmol) in DCM (2 mL) zugetropft. Das Reaktionsgemisch wurde für 3 h bei 40 °C gerührt, bis die Lösung grün gefärbt war. Das Lösungsmittel wurde unter Vakuum entfernt und der braun-grüne Rückstand in Pentan/DCM (2:1) aufgenommen und über Celite filtriert. Das Lösungsmittel wurde eingeengt und die braun-grüne Lösung säulenchromatographisch an Kieselgel (Lösungsmittel: erst DCM, dann EtOAc) gereinigt. Der geschützte Grubbs-Hoveyda Katalysator der zweiten Generation ([1,3-bis(1-mesityl)-4-tetrahydropyranyl-4,5-dihydroimidazol-2-yliden][2-isopropoxybenzyliden]ruthenium(IV)-dichlorid) wurde als grüner Feststoff (550 mg, 0,74 mmol) erhalten.

Eine Lösung aus [1,3-bis(1-mesityl)-4-tetrahydropyranyl-4,5-dihydroimidazol-2-yliden][2-isopropoxy-benzyliden]-ruthenium(IV)-dichlorid (550 mg, 0,74 mmol) in Ethanol (20 mL) wurde mit wässriger HCl-Lösung (1,0 M, 0,30 mL) versetzt und für 20 min mit Argon entgast. Die Lösung wurde unter Argonatmosphäre für 72 h gerührt. Das Lösungsmittel wurde unter Vakuum entfernt. Der grüne Rückstand wurde in DCM (20 mL) aufgenommen und mit Wasser (2 × 20 mL) gewaschen. Die org. Phase wurde über Na₂SO₄ getrocknet, das Lösungsmittel unter Vakuum entfernt und der grün-braune Rückstand säulenchromatographisch an Kieselgel (Lösungsmittel: DCM) gereinigt. Der Grubbs-Hoveyda Katalysator der zweiten Generation ([1,3-Bis(1-mesityl)-4-methylhydroxy-4,5-dihydroimidazol-2-yliden][2-isopropoxybenzyliden]ruthenium(IV)-dichlorid) konnte als grüner Feststoff (350 mg, 0,533 mmol) erhalten werden.

([1,3-Bis(1-mesityl)-4-methylhydroxy-4,5-dihydroimidazol-2-yliden][2-isopropoxybenzyliden]ruthenium(IV)-dichlorid) (30 mg, 0,05 mmol) wurde in THF (0,5 mL) vorgelegt. Eine äquimolare Menge des *N*-Maleimidopropansäurechlorids (13 mg, 0,05 mmol) wurde in THF (0,5 mL) zugetropft. Zu der Lösung wurde NaHCO₃ (30 mg, 0,35 mmol) zugegeben. Das Reaktionsgemisch wurde für 24 h bei 25 °C gerührt. Anschließend wurde die Lösung filtriert und mit Et₂O (15 mL) verdünnt. Die Lösung wurde mit Wasser (3 x 15 mL) gewaschen und die org. Phase über Na₂SO₄ getrocknet und das Lösungsmittel unter Vakuum entfernt. Der Grubbs-Hoveyda Katalysator der zweiten Generation mit Maleimid-Gruppe ([1,3-Bis(1-mesityl)-4-(methyl-N-maleimidopropansäure-ester)-4,5-dihydroimidazol-2-yliden][2-isopropoxybenzyliden] ruthenium(IV)-dichlorid) wurde als grüner Feststoff (35 mg, 0,047 mmol) erhalten. (Vgl. hierzu Philippart, F., Arlt, M., Gotzen, S., Tenne, S.-J., Bocola, M., Chen, H.-H., Zhu, L., Schwaneberg, U. und Okuda, J., A Biohybrid Ring-Opening Metathesis Polymerization Catalyst Based on an Engineered Variant of the β-Barrel Protein FhuA, Chem. Eur. J., 2013, 19, 13865-13871.)

### 2. Bereitstellung des Expressionsstamms Pseudomonas putida:

Der Expressionsstamm *P. putida* KT2440 wurde erhalten von ATCC.

### 3. Design und Herstellung des Proteingerüsts (hier: FhuA ΔCVF^{TEV} (SEQ ID No: 5; SEQ ID No: 6):

Ausgehend vom FhuA WT (pdb: 1BY3, Locher, K. P.; Rees, B.; Koebnik, R.; Mitschier, A.; Moulinier, L.; Rosenbusch, J. P.; Moras, D., , Cell 95 (1998) 771-778) wurden die Aminosäuren 1-60 deletiert und an Position 545 Lysin gegen Cystein ausgetauscht (Nallani, M.; Onaca, O.; Gera, N.; Hildenbrand, K.; Hoheisel, W.; Schwaneberg, U., Biotechnology Journal 1 (2006) 828-834). Durch Austausch von Asparagin zu Valin an Position 548 und Glutaminsäure zu Phenylalanin an Position 501 konnten Cystein-Zugänglichkeit und Katalysatorkompatibilität verbessert werden. Die TEV-Schnittstellen ENLYFQ|G wurden eingefügt in den Loops 7 (zwischen Asp509 und Lys508) und 8 (zwischen Asn548 und Glycine549). Die Expression von FhuA ΔCVF^{TEV} in der äußeren Membran von *P. putida* wurde erreicht durch Entfernen der nativen *E. coli* FhuA-Signalsequenz und Einführen einer *P*. *putida* Signalsequenz (24 Aminosäuren) von Porin F mittels PLICing. (SEQ ID No: 17 (MKLKNTLGVVIGSLVAASAMNAFA); SEQ ID No: 18 (ATG AAA CTG AAG AAC ACC TTA GGC GTT GTC ATC GGC TCG CTG GTT GCC GCT TCG GCA ATG AAC GCC TTC GCC)).

Das synthetische Gen mit den oben beschriebenen Modifikationen wurde mittels Restriktionsklonierung (EcoRI und Ndel) in den Expressionsvektor pUCP-Ndel eingeführt. FhuA wurde in den Expressionswirt *P*. *putida* KT2440 mittels Elektroporation transformiert. Die Expression wurde durchgeführt in LB-Medium, enthaltend 1 mM Ampicillin bei 37 °C für 12 h mit Isopropyl-β-D-1-thiogalactopyranosid (IPTG, 1 mM), um die Expression zu induzieren. Nach Übernachtexpression wurde die Kultur durch Zentrifugation geerntet.

### 4. Anbinden der katalytisch aktiven Metallkomplexe an das Proteingerüst:

Das geerntete Pellet wurde mit ddH₂O/Glyzerin gewaschen und in flüssigem Stickstoff eingefroren. Die Zellen wurden anschließend lyophilisiert und in PBS-Puffer (pH 7,5) rehydriert. Die jeweiligen Metallkomplexe (s.o., Bsp. 1.) wurden *via* Maleimid und Cystein gekoppelt mit 10 Äquivalenten Katalysator in PBS-Puffer (pH 7,5) und 20% (v/v) THF als Co-Lösungsmittel. Die Suspension wurde 24 h bei Raumtemperatur gerührt, gereinigt, zentrifugiert (4000 rpm, 10 min) und mit PBS/THF (1:1) mindestens 5 Mal gewaschen. Die Waschlösungen wurden mithilfe von HPLC-Chromatographie untersucht, um die Entfernung von nicht gebundenem Katalysator zu bestätigen. (Als Negativkontrolle wurde als Proteingerüst die Variante FhuA Δ1-160 (SEQ ID No: 7; SEQ ID No: 8) ohne frei verfügbares Cystein verwendet.)

### 5. Katalytische Reaktion mit erfindungsgemäßen Biohybridkatalysator-Systemen:

### Metathese (ROMP, RCM, CM):

Für die ROMP wurden 5 ml einer Zellsuspension aus Biohybridkatalysator-Systemen mit Grubbs-Hoveyda-II-Katalysator mit Maleimid-Gruppe (s.o., Bsp. 1.) in 10 mM NaPi (pH 5,8) mit 20% (v/v) THF, entsprechend einer molaren Katalysatorladung von 0,1 Mol%, bereitgestellt und mit 15 µl Monomer (7-Oxanorbonen) versetzt. Nach 68 h Rühren bei 25 °C wurden die Produkte isoliert und mittels NMR-Spektroskopie identifiziert.

### Beispiel 3: E. coli Biohybridkatalysator-Systeme mit LCI als Proteingerüst

### 1. Synthese katalytisch aktiver Metallkomplexe:

### 1.a) Synthese von ([1,3-Bis(1-mesityl)-4-(methyl-N-maleimidopropansäure-ester)-4,5-dihydroimidazol-2-yliden][2-isopropoxybenzyliden]-ruthenium(IV)-dichlorid) [Grubbs-Hoveyda-II mit Maleimid-Gruppe]:

1,3-Dimesityl-4,5-dihydro-4-(tetrahydropyranyl)-1*H*-imidazol-3-ium-chlorid (503 mg, 1,10 mmol) wurde in THF (5 mL) aufgenommen und mit Kaliumhexamethyldisilazid (220 mg, 1,10 mmol) versetzt. Diese Lösung wurde zu einer Lösung des Grubbs-Katalysator der ersten Generation (823 mg, 1,00 mmol, 1,00 Äquiv.) in THF (10 mL) zugefügt. Das Reaktionsgemisch wurde für 16 h bei 60 °C gerührt. Das Lösungsmittel wurde unter Vakuum entfernt, der rote Rückstand in Benzol (3 mL) aufgenommen und säulenchromatographisch an Kieselgel gereinigt (Lösungsmittel: n-Pentan/Et₂O - 2:1). Der Grubbs-Katalysator der zweiten Generation (Tricyclohexylphosphin[1,3-bis(1-mesityl)-4-tetrahydropyranyl-4,5-dihydroimidazol-2-yliden][benzyliden]ruthenium(IV)-dichlorid) wurde als roter Feststoff erhalten.

Tricyclohexylphosphin[1,3-bis(1-mesityl)-4-tetrahydropyranyl-4,5-dihydroimidazol-2-yliden][benzyliden]ruthenium(IV)-dichlorid (860 mg, 0,891 mmol) und Kupfer(I)-Chlorid (133 mg, 1,34 mmol) wurden in DCM (8 mL) aufgenommen. Zu der roten Lösung wurde eine Lösung aus 2-Isopropoxystyrol (182 mg, 1,13 mmol) in DCM (2 mL) zugetropft. Das Reaktionsgemisch wurde für 3 h bei 40 °C gerührt, bis die Lösung grün gefärbt war. Das Lösungsmittel wurde unter Vakuum entfernt und der braun-grüne Rückstand in Pentan/DCM (2:1) aufgenommen und über Celite filtriert. Das Lösungsmittel wurde eingeengt und die braun-grüne Lösung säulenchromatographisch an Kieselgel (Lösungsmittel: erst DCM, dann EtOAc) gereinigt. Der geschützte Grubbs-Hoveyda Katalysator der zweiten Generation ([1,3-bis(1-mesityl)-4-tetrahydropyranyl-4,5-dihydroimidazol-2-yliden][2-isopropoxybenzyliden]ruthenium(IV)-dichlorid) wurde als grüner Feststoff (550 mg, 0,74 mmol) erhalten.

Eine Lösung aus [1,3-bis(1-mesityl)-4-tetrahydropyranyl-4,5-dihydroimidazol-2-yliden][2-isopropoxybenzyliden]-ruthenium(IV)-dichlorid (550 mg, 0,74 mmol) in Ethanol (20 mL) wurde mit wässriger HCl-Lösung (1,0 M, 0,30 mL) versetzt und für 20 min mit Argon entgast. Die Lösung wurde unter Argonatmosphäre für 72 h gerührt. Das Lösungsmittel wurde unter Vakuum entfernt. Der grüne Rückstand wurde in DCM (20 mL) aufgenommen und mit Wasser (2 × 20 mL) gewaschen. Die org. Phase wurde über Na₂SO₄ getrocknet, das Lösungsmittel unter Vakuum entfernt und der grün-braune Rückstand säulenchromatographisch an Kieselgel (Lösungsmittel: DCM) gereinigt. Der Grubbs-Hoveyda Katalysator der zweiten Generation ([1,3-Bis(1-mesityl)-4-methylhydroxy-4,5-dihydroimidazol-2-yliden][2-isopropoxybenzyliden]ruthenium(IV)-dichlorid) konnte als grüner Feststoff (350 mg, 0,533 mmol) erhalten werden.

([1,3-Bis(1-mesityl)-4-methylhydroxy-4,5-dihydroimidazol-2-yliden][2-isopropoxybenzyliden]ruthenium(IV)-dichlorid) (30 mg, 0,05 mmol) wurde in THF (0,5 mL) vorgelegt. Eine äquimolare Menge des *N*-Maleimidopropansäurechlorid (13 mg, 0,05 mmol) wurde in THF (0,5 mL) zugetropft. Zu der Lösung wurde NaHCO₃ (30 mg, 0,35 mmol) zugegeben. Das Reaktionsgemisch wurde für 24 h bei 25 °C gerührt. Anschließend wurde die Lösung filtriert und mit Et₂O (15 mL) verdünnt. Die Lösung wurde mit Wasser (3 × 15 mL) gewaschen und die org. Phase über Na₂SO₄ getrocknet und das Lösungsmittel unter Vakuum entfernt. Der Grubbs-Hoveyda Katalysator der zweiten Generation mit Maleimid-Gruppe ([1,3-Bis(1-mesityl)-4-(methyl-N-maleimidopropansäure-ester)-4,5-dihydroimidazol-2-yliden][2-isopropoxybenzyliden] ruthenium(IV)-dichlorid) wurde als grüner Feststoff (35 mg, 0,047 mmol) erhalten. (Vgl. hierzu Philippart, F., Arlt, M., Gotzen, S., Tenne, S.-J., Bocola, M., Chen, H.-H., Zhu, L., Schwaneberg, U. und Okuda, J., A Biohybrid Ring-Opening Metathesis Polymerization Catalyst Based on an Engineered Variant of the β-Barrel Protein FhuA, Chem. Eur. J., 2013, 19, 13865-13871.)

### 1. b) Synthese von Rh-1,5-cyclooctadienyl-cyclopentadienyl-ethyl-maleimid [RhCOD-Katalysator]:

Cyclooctadien Rhodium Chlorid Dimer [Rh(cod)Cl]₂ (79 mg, 0,16 mmol) wird in THF (3 mL) vorgelegt. Zu dieser Lösung wird eine Lösung aus 2-(cyclopentadienyl)-ethan-1-amin Lithium C₇H₁₀NLi (40 mg, 0,37 mmol) in 3 mL THF zugetropft. Die Lösung wird für 30 Minuten bei 25 °C gerührt. Anschließend wird N-methyoxcarbonylmaleimid (C₆H₅NO₄) (55 mg, 0,37 mmol) in 10 mL THF langsam zugetropft, und die Reaktionsmischung für 24 Stunden bei 25 °C gerührt. Nach beendeter Reaktion wird Wasser (30 mL) zugegeben, und die Lösung mit DCM (3 x 30 mL) ausgeschüttelt. Das organische Lösungsmittel wird im Vakuum entfernt und der Rückstand säulenchromatographisch aufgereinigt (Al₂O₃, THF). Der Rh-Katalysator (C₂₁H₂₆NO₂Rh) wird als oranges Pulver (ca. 50 mg) erhalten. (Vgl. hierzu Onoda, A., Fukumoto, K., Arlt, M., Bocola, M., Schwaneberg U., Hayashi, T., A rhodium complex-linked β-barrel protein as a hybrid biocatalyst for phenylacetylene polymerization", Chem. Comm., 2012, 48, 9756-9758.)

### 2. Bereitstellung des Expressionsstamms E. coli BL21 (DF-3):

### E. coli BL21 (DE3) wurde erhalten von Novagen.

### 3. Design und Herstellung des Proteingerüsts (hier: Cys-LCI (SEQ ID No: 9; SEQ ID No: 10):

LCI-Cys wurde als synthetisches Gen basierend auf der Kristallstruktur 2B9K mit einem zusätzlichen freien Cystein am N-terminus erhalten. Die Darstellung des LCI-Cys an der Zelloberfläche wurde erreicht durch ein EstA Autotransporter System (Becker, S., Theile, S., Heppeler, N., Michalczyk, A., Wentzel, A., Wilhelm, S., Jaeger, K.-E., and Kolmar, H. (2005). FEBS Lett. 579, 1177-1182.). EstA besteht aus einer N-terminalen Domäne mit Esteraseaktivität und der C-terminalen Domäne, die eine fassartige Struktur durch die äußere Bakterienmembran bildet. Die C-terminale Domäne reguliert die Translokation der Esteraseaktivität an die Zelloberfläche. Durch Deaktivierung der Esteraseaktivität und Modifizierung des N-Terminus mit LCI-Cys und einem 14 Aminosäurelinker kann die Zelloberflächenpräsentation des LCI-Cys ermöglicht werden (csd-LCI-Cys). Mittels PLICing wurde die EstA-Sequenz in das LCI-Cys-Plasmid eingefügt.
Die Expression wurde durchgeführt in LB-Medium, enthaltend 1 mM Ampicillin, als Vorkultur und TB als Hauptkultur bei 37 °C für 12 h mit Isopropyl-β-D-1-thiogalactopyranosid (IPTG, 1 mM), um die Expression zu induzieren. Nach Übernachtexpression wurde die Kultur durch Zentrifugation geerntet.

### 4. Anbinden der katalytisch aktiven Metallkomplexe an das Proteingerüst:

Das geerntete Pellet wurde mit ddH₂O/Glyzerin gewaschen und in flüssigem Stickstoff eingefroren. Die Zellen werden anschließend lyophilisiert und in Puffer (20 mM Tris, 100 mM NaCl, pH 8) rehydriert. Die jeweiligen Metallkomplexe werden *via* Maleimid und Cystein gekoppelt mit 10 Äquivalenten Katalysator in PBS-Puffer (pH 7,5) und 20% (v/v) THF als Co-Lösungsmittel. Die Suspension wird 24 h bei Raumtemperatur gerührt, gereinigt durch Zentrifugation (4000 rpm, 10 min) und mit Puffer/THF (5:1) mindestens 3 Mal und mit reinem Puffer mindestens 2 Mal gewaschen. Die Waschlösungen werden mit Hilfe von HPLC-Chromatographie untersucht, um die Entfernung von nicht gebundenem Katalysator zu bestätigen. (Als Negativkontrolle wird die Variante LCI ohne frei verfügbares Cystein (SEQ ID No: 11; SEQ ID No: 12) verwendet.)

### 5. Katalytische Reaktion mit erfindungsgemäßen Biohybridkatalysator-Systemen:

### 5.a) Phenylacetylen-Polymerisierung und -Trimerisierung:

Für die Phenylacetylen-Polymerisierung wurden 5 ml einer Zellsuspension aus Biohybridkatalysator-Systemen mit RhCOD-Katalysator (s.o., Bsp. 1.b) in 10 mM NaPi (pH 8), entsprechend einer molaren Katalysatorladung von 0,1 Mol%, bereitgestellt und mit 15 µl Monomer (Phenylacetylen) versetzt. Nach 48 h Rühren bei 25 °C wurden die Produkte isoliert und mittels NMR-Spektroskopie identifiziert.

### 5.b) Metathese (ROMP, RCM, CM):

Für die ROMP wurden 5 ml einer Zellsuspension aus Biohybridkatalysator-Systemen mit Grubbs-Hoveyda-II-Katalysator mit Maleimid-Gruppe (s.o., Bsp. 1.a) in 10 mM NaPi (pH 5,8) mit 20% (v/v) THF, entsprechend einer molaren Katalysatorladung von 0,1 Mol%, bereitgestellt und mit 15 µl Monomer (7-Oxanorbonen) versetzt. Nach 68 h Rühren bei 25 °C wurden die Produkte isoliert und mittels NMR-Spektroskopie identifiziert.

### Beispiel 4: E. coli Biohybridkatalysator-Systeme mit csdNitrobindin-NB4 als Proteingerüst

### 1. Synthese katalytisch aktiver Metallkomplexe:

### 1.a) Synthese von ([1,3-Bis(1-mesityl)-4-(methyl-N-maleimidopropansäure-ester)-4,5-dihydroimidazol-2-yliden][2-isopropoxybenzyliden] ruthenium(IV)-dichlorid) [Grubbs-Hoveyda-II mit Maleimid-Gruppe]:

1,3-Dimesityl-4,5-dihydro-4-(tetrahydropyranyl)-1*H*-imidazol-3-ium-chlorid (503 mg, 1,10 mmol) wurde in THF (5 mL) aufgenommen und mit Kaliumhexamethyldisilazid (220 mg, 1,10 mmol) versetzt. Diese Lösung wurde zu einer Lösung des Grubbs-Katalysator der ersten Generation (823 mg, 1,00 mmol, 1,00 Äquiv.) in THF (10 mL) zugefügt. Das Reaktionsgemisch wurde für 16 h bei 60 °C gerührt. Das Lösungsmittel wurde unter Vakuum entfernt, der rote Rückstand in Benzol (3 mL) aufgenommen und säulenchromatographisch an Kieselgel gereinigt (Lösungsmittel: n-Pentan/Et₂O - 2:1). Der Grubbs-Katalysator der zweiten Generation (Tricyclohexylphosphin[1,3-bis(1-mesityl)-4-tetrahydropyranyl-4,5-dihydroimidazol-2-yliden][benzyliden]ruthenium(IV)-dichlorid) wurde als roter Feststoff erhalten.

Tricyclohexylphosphin[1,3-bis(1-mesityl)-4-tetrahydropyranyl-4,5-dihydroimidazol-2-yliden][benzyliden]ruthenium(IV)-dichlorid (860 mg, 0,891 mmol) und Kupfer(I)-Chlorid (133 mg, 1,34 mmol) wurden in DCM (8 mL) aufgenommen. Zu der roten Lösung wurde eine Lösung aus 2-Isopropoxystyrol (182 mg, 1,13 mmol) in DCM (2 mL) zugetropft. Das Reaktionsgemisch wurde für 3 h bei 40 °C gerührt, bis die Lösung grün gefärbt war. Das Lösungsmittel wurde unter Vakuum entfernt und der braun-grüne Rückstand in Pentan/DCM (2:1) aufgenommen und über Celite filtriert. Das Lösungsmittel wurde eingeengt und die braun-grüne Lösung säulenchromatographisch an Kieselgel (Lösungsmittel: erst DCM, dann EtOAc) gereinigt. Der geschützte Grubbs-Hoveyda Katalysator der zweiten Generation ([1,3-bis(1-mesityl)-4-tetrahydropyranyl-4,5-dihydroimidazol-2-yliden][2-isopropoxybenzyliden]ruthenium(IV)-dichlorid) wurde als grüner Feststoff (550 mg, 0,74 mmol) erhalten. Eine Lösung aus [1,3-bis(1-mesityl)-4-tetrahydropyranyl-4,5-dihydroimidazol-2-yliden][2-isopropoxybenzyliden]ruthenium(IV)-dichlorid (550 mg, 0,74 mmol) in Ethanol (20 mL) wurde mit wässriger HCl-Lösung (1,0 M, 0,30 mL) versetzt und für 20 min mit Argon entgast. Die Lösung wurde unter Argonatmosphäre für 72 h gerührt. Das Lösungsmittel wurde unter Vakuum entfernt. Der grüne Rückstand wurde in DCM (20 mL) aufgenommen und mit Wasser (2 x 20 mL) gewaschen. Die org. Phase wurde über Na₂SO₄ getrocknet, das Lösungsmittel unter Vakuum entfernt und der grün-braune Rückstand säulenchromatographisch an Kieselgel (Lösungsmittel: DCM) gereinigt. Der Grubbs-Hoveyda Katalysator der zweiten Generation ([1,3-Bis(1-mesityl)-4-methylhydroxy-4,5-dihydroimidazol-2-yliden][2-isopropoxybenzyliden]-ruthenium (IV)-dichlorid) konnte als grüner Feststoff (350 mg, 0,533 mmol) erhalten werden.

([1,3-Bis(1-mesityl)-4-methylhydroxy-4,5-dihydroimidazol-2-yliden][2-isopropoxybenzyliden]ruthenium(IV)-dichlorid) (30 mg, 0,05 mmol) wurde in THF (0,5 mL) vorgelegt. Eine äquimolare Menge des *N*-Maleimidopropansäurechlorids (13 mg, 0,05 mmol) wurde in THF (0,5 mL) zugetropft. Zu der Lösung wurde NaHCO₃ (30 mg, 0,35 mmol) zugegeben. Das Reaktionsgemisch wurde für 24 h bei 25 °C gerührt. Anschließend wurde die Lösung filtriert und mit Et₂O (15 mL) verdünnt. Die Lösung wurde mit Wasser (3 x 15 mL) gewaschen und die org. Phase über Na₂SO₄ getrocknet und das Lösungsmittel unter Vakuum entfernt. Der Grubbs-Hoveyda Katalysator der zweiten Generation mit Maleimid-Gruppe ([1,3-Bis(1-mesityl)-4-(methyl-*N*-maleimidopropansäure-ester)-4,5-dihydroimidazol-2-yliden][2-isopropoxybenzyliden] ruthenium(IV)-dichlorid) wurde als grüner Feststoff (35 mg, 0,047 mmol) erhalten. (Vgl. hierzu Philippart, F., Arlt, M., Gotzen, S., Tenne, S.-J., Bocola, M., Chen, H.-H., Zhu, L., Schwaneberg, U. und Okuda, J., A Biohybrid Ring-Opening Metathesis Polymerization Catalyst Based on an Engineered Variant of the β-Barrel Protein FhuA, Chem. Eur. J., 2013, 19, 13865-13871.)

### 1. b) Synthese von Rh-1,5-cyclooctadienyl-cyclopentadienyl-ethyl-maleimid [RhCOD-Katalysator]:

Cyclooctadien Rhodium Chlorid Dimer [Rh(cod)Cl]₂ (79 mg, 0,16 mmol) wird in THF (3 mL) vorgelegt. Zu dieser Lösung wird eine Lösung aus 2-(cyclopentadienyl)-ethan-1-amin Lithium C₇H₁₀NLi (40 mg, 0,37 mmol) in 3 mL THF zugetropft. Die Lösung wird für 30 Minuten bei 25 °C gerührt. Anschließend wird N-methyoxcarbonylmaleimid (C₆H₅NO₄) (55 mg, 0,37 mmol) in 10 mL THF langsam zugetropft, und die Reaktionsmischung für 24 Stunden bei 25 °C gerührt. Nach beendeter Reaktion wird Wasser (30 mL) zugegeben, und die Lösung mit DCM (3 x 30 mL) ausgeschüttelt. Das organische Lösungsmittel wird im Vakuum entfernt und der Rückstand säulenchromatographisch aufgereinigt (Al₂O₃, THF). Der Rh-Katalysator (C₂₁H₂₆NO₂Rh) wird als oranges Pulver (ca. 50 mg) erhalten. (Vgl. hierzu Onoda, A., Fukumoto, K., Arlt, M., Bocola, M., Schwaneberg U., Hayashi, T., A rhodium complex-linked β-barrel protein as a hybrid biocatalyst for phenylacetylene polymerization", Chem. Comm., 2012, 48, 9756-9758.)

### 2. Bereitstellung des Expressionsstamms E. coli BL21(DE3):

### E. coli BL21 (DE3) wurde erhalten von Novagen.

### 3. Design und Herstellung des Proteingerüsts (hier: csd-NB4 (SEQ ID No: 13; SEQ ID No: 14):

Das Nitrobindin-NB4 Biohybridkatalysatorgerüst wurde erhalten von Fukumoto *et al.* (K. Fukumoto, A. Onoda, E. Mizohata, M. Bocola, T. Inoue, U. Schwaneberg, T. Hayashi, ChemCatChem. (2014), 6, 1229-1235). Die Darstellung des NB4 an der Zelloberfläche wurde erreicht durch ein EstA Autotransporter System (Becker, S., Theile, S., Heppeler, N., Michalczyk, A., Wentzel, A., Wilhelm, S., Jaeger, K.-E., and Kolmar, H. (2005). FEBS Lett. 579, 1177-1182.). EstA besteht aus einer N-terminalen Domäne mit Esteraseaktivität und der C-terminalen Domäne, die eine fassartige Struktur durch die äußere Bakterienmembran bildet. Die C-terminale Domäne reguliert die Translokation der Esteraseaktivität an die Zelloberfläche. Durch Deaktivierung der Esteraseaktivität und Modifizierung des N-Terminus mit NB-4, kann die Zelloberflächenpräsentation des NB4 ermöglicht werden (csd-NB4). Mittels Restriktionsverdau und anschließender Ligation wurde die NB4-Sequenz in das EstA-Plasmid eingefügt. Die Expression wurde durchgeführt in LB-Medium, enthaltend 1 mM Ampicillin, als Vorkultur und LB als Hauptkultur bei 18 °C für 12 h mit Isopropyl-β-D-1-thiogalactopyranosid (IPTG, 1 mM), um die Expression zu induzieren. Nach Übernachtexpression wurde die Kultur durch Zentrifugation geerntet.

### 4. Anbinden der katalytisch aktiven Metallkomplexe an das Proteingerüst:

Die geernteten Zellen wurden bei -20°C eingefroren, anschließend lyophilisiert und in Puffer (20 mM Tris, 100 mM NaCl, pH 8) rehydriert. Die Metallkomplexe wurden *via* Maleimid und Cystein gekoppelt mit 100 Äquivalenten Katalysator in PBS-Puffer (pH 7,5) und 20% (v/v) THF als Co-Lösungsmittel. Die Suspension wurde 30 min bei Raumtemperatur gerührt, gereinigt durch Zentrifugation (4000 rpm, 10 min) und mit Puffer/THF (1:1) mindestens 3 Mal und mit Puffer mindestens 2 Mal gewaschen. (Als Negativkontrolle wurde die Variante Nitrobindin ohne frei verfügbares Cystein (SEQ ID No: 15; SEQ ID No: 16) verwendet.)

### 5. Katalytische Reaktion mit erfindungsgemäßen Biohybridkatalysator-Systemen:

### 5.a) Phenylacetylen-Polymerisierung und -Trimerisierung:

Für die Phenylacetylen-Polymerisierung wurden 5 ml einer Zellsuspension aus Biohybridkatalysator-Systemen mit RhCOD-Katalysator (s.o., Bsp. 1.b) in 10 mM NaPi (pH 8), entsprechend einer molaren Katalysatorladung von 0,1 Mol%, bereitgestellt und mit 1M Monomer (Phenylacetylen) versetzt. Nach 12 h Rühren bei 25 °C wurden die Produkte isoliert und mittels NMR-Spektroskopie identifiziert.

### 5.b) Metathese (ROMP, RCM, CM):

Für die ROMP wurden 5 ml einer Zellsuspension aus Biohybridkatalysator-Systemen mit Grubbs-Hoveyda-II-Katalysator mit Maleimid-Gruppe (s.o., Bsp. 1.a) in 10 mM NaPi (pH 5,8) mit 20% (v/v) THF, entsprechend einer molaren Katalysatorladung von 0,1 Mol%, bereitgestellt und mit 15 µl Monomer (7-Oxanorbonen) versetzt. Nach 68 h Rühren bei 25 °C wurden die Produkte isoliert und mittels NMR-Spektroskopie identifiziert.

## Patentansprüche

1. Biohybridkatalysator-System, umfassend oder bestehend aus
(a) einer intakten, nicht-menschlichen Zelle und
(b) einem oder mehreren künstlichen Metalloenzym(en) bestehend aus
(b1) einem Proteingerüst und
(b2) einem an das Proteingerüst angebundenen, katalytisch aktiven Metallkomplex, enthaltend ein Übergangsmetall, vorzugsweise ein Metall ausgewählt aus der Gruppe bestehend aus Ruthenium, Rhodium, Cobalt, Kupfer, Iridium, Mangan, Palladium und Platin,
wobei das/die Metalloenzym(e) in der Zellmembran verankert und/oder an der Zelloberfläche der Zelle angebunden ist/sind und
wobei der Metallkomplex ausgewählt ist aus der Gruppe bestehend aus organometallischen Katalysatoren, vorzugsweise Grubbs-Hoveyda-Katalysatoren, Rh-Cyclopentadien-Katalysatoren, Rh-Carben-Katalysatoren, Cu-Carben-Katalysatoren, Cu-Terpyridin-Katalysatoren, Cu-Phenanthrolin-Katalysatoren, Cu-Pyren-Katalysatoren, Co-Terpyridin-Katalysatoren, Pt-Terpyridin-Katalysatoren, Pt-Carben-Katalysatoren, Pd-Terpyridin-Katalysatoren, Pd-Carben-Katalysatoren, Ir-Carben-Katalysatoren und Mn-Cyclopentadienyl-Katalysatoren.

2. Biohybridkatalysator-System nach Anspruch 1, wobei die Zelle ausgewählt ist aus der Gruppe bestehend aus Bakterienzellen, vorzugsweise gram-negativen Bakterienzellen.

3. Biohybridkatalysator-System nach Anspruch 1 oder 2, wobei das Proteingerüst ausgewählt ist aus der Gruppe bestehend aus Transmembranproteinen und löslichen Proteinen, vorzugsweise solchen mit beta-Fassstruktur, vorzugsweise solchen mit 6 bis 24 beta-Faltblättern, bevorzugt FhuA oder Nitrobindin, membranständigen oder verankerten Proteinen, vorzugsweise LCI.

4. Biohybridkatalysator-System nach einem der vorangehenden Ansprüche, wobei das/die Metalloenzym(e) über Zelloberflächendisplay an der Zelloberfläche der Zelle angebunden ist/sind.

5. Verfahren zur Herstellung eines Biohybridkatalysator-Systems nach einem der vorangehenden Ansprüche, umfassend folgende Schritte:
(ii.1) Bereitstellen eines Proteingerüsts, das Bestandteil einer intakten, nicht-menschlichen Zelle, vorzugsweise Bestandteil der Zellmembran und/oder der Zelloberfläche einer intakten, nicht-menschlichen Zelle ist, und
(ii.2) Anbinden eines katalytisch aktiven Metallkomplexes, enthaltend ein Übergangsmetall, vorzugsweise ein Metall ausgewählt aus der Gruppe bestehend aus Ruthenium, Rhodium, Cobalt, Kupfer, Iridium, Mangan, Palladium und Platin, an das Proteingerüst, vorzugsweise mithilfe dativer oder supramolekularer Wechselwirkungen oder mithilfe kovalenter Bindungen,
wobei der Metallkomplex in Schritt (ii.2) ausgewählt ist aus der Gruppe bestehend aus organometallischen Katalysatoren, vorzugsweise Grubbs-Hoveyda-Katalysatoren, Rh-Cyclopentadien-Katalysatoren, Rh-Carben-Katalysatoren, Cu-Carben-Katalysatoren, Cu-Terpyridin-Katalysatoren, Cu-Phenanthrolin-Katalysatoren, Cu-Pyren- und Co-Terpyridin-Katalysatoren, Pt-Terpyridin-Katalysatoren, Pt-Carben-Katalysatoren, Pd-Terpyridin-Katalysatoren, Pd-Carben-Katalysatoren, Ir-Carben-Katalysatoren und Mn-Cyclopentad ienyl-Katalysatoren.

6. Verfahren nach Anspruch 5, wobei die Zelle in Schritt (ii.1) ausgewählt ist aus der Gruppe bestehend aus Bakterienzellen, vorzugsweise gram-negativen Bakterienzellen.

7. Verfahren nach Anspruch 5 oder 6, wobei das Proteingerüst in Schritt (ii.1) ausgewählt ist aus der Gruppe bestehend aus Transmembranproteinen und löslichen Proteinen, vorzugsweise solchen mit beta-Fassstruktur, vorzugsweise solchen mit 6 bis 24 beta-Faltblättern, bevorzugt FhuA oder Nitrobindin, membranständigen oder verankerten Proteinen, vorzugsweise LCI.

8. Biohybridkatalysator-System nach einem der Ansprüche 1 bis 4, hergestellt oder herstellbar durch ein Verfahren nach einem der Ansprüche 5 bis 7.

9. Verfahren zur Katalyse einer chemischen Reaktion, umfassend folgende Schritte:
(a) Bereitstellen eines Biohybridkatalysator-Systems nach einem der Ansprüche 1 bis 4 oder 8, und
(b) Katalyse einer chemischen Reaktion unter Verwendung des Biohybridkatalysator-Systems, wobei die Reaktion durch die katalytische Aktivität des an das Proteingerüst angebundenen, katalytisch aktiven Metallkomplexes katalysiert wird.

10. Verfahren nach Anspruch 9, wobei die chemische Reaktion ausgewählt ist aus der Gruppe bestehend aus Metathese, Acetylen-Trimerisierung und -Polymerisierung, Diels-Alder-Reaktionen, Hydrogenierungen und Lactidpolymerisationen.

11. Verfahren nach Anspruch 9 oder 10, zudem umfassend, vorzugsweise zwischen Schritt (a) und Schritt (b), folgenden Schritt:
- Immobilisierung der intakten, nicht-menschlichen Zelle des Biohybridkatalysator-Systems gemäß einem der Ansprüche 1 bis 4 oder 8 an der Oberfläche eines Objekts.

12. Verfahren nach einem der Ansprüche 9 bis 11, zudem umfassend folgenden Schritt:
- Abtrennen des Biohybridkatalysator-Systems von dem/den Reaktionsprodukt(en) der chemischen Reaktion.

13. Verwendung eines Biohybridkatalysator-Systems nach einem der Ansprüche 1 bis 4 oder 8 als Katalysator zur Katalyse einer chemischen Reaktion, vorzugsweise zur Katalyse einer Reaktion wie in Anspruch 10 definiert.
